# EUROPEAN PATENT SPECIFICATION

(11) **EP 4 220 655 B1**
(45) Date of publication and mention of the grant of the patent: **08.07.2026**
(21) Application number: 23153716.8
(22) Date of filing: 27.01.2023
(51) Int. Cl.: G16H 20/30, G16H 40/63, G16H 40/67, G16H 50/30

(54) **INFORMATION PROCESSING METHOD, RECORDING MEDIUM, AND INFORMATION PROCESSING SYSTEM**
INFORMATIONSVERARBEITUNGSVERFAHREN, AUFZEICHNUNGSMEDIUM UND INFORMATIONSVERARBEITUNGSSYSTEM
PROCÉDÉ DE TRAITEMENT D'INFORMATIONS, SUPPORT D'ENREGISTREMENT ET SYSTÈME DE TRAITEMENT D'INFORMATIONS

(30) Priority: 31.01.2022 JP 2022012591
(43) Date of publication of application: 02.08.2023
(73) Proprietor: Casio Computer Co., Ltd., Tokyo 151-8543 (JP)
(72) Inventor: FUKUSHIMA, Reina, Tokyo, 205-8555 (JP); YAMAMOTO, Futoshi, Tokyo, 205-8555 (JP)
(74) Representative: Plougmann Vingtoft a/s

(56) References cited:
- JP-A- 2020 150 987

## Description

### Technical Field

The present invention relates to an information processing method, a recording medium, and an information processing system.

### Background Art

Conventionally, there is a technique in which the activity state of a subject, such as a test subject, is detected by a wearable device worn on the subject, and the activity state is evaluated based on the analysis result of the detected activity state. For example, JP 2020-150987 A discloses a technique for presenting advice on the form of a certain activity to a subject having performed the certain activity based on the analysis result of the activity state of the subject. **In** addition, JP 2020-150987 A discloses a technique for deriving and presenting a rank of each object in a plurality of subjects having performed the certain activity based on the analysis results of the activity states of the plurality of obj ects. The subject can improve the skill related to activity by improving the activity state with reference to the advice and rank.

However, when only one activity state related to activity is evaluated, there is a bias in skills improved by activity, and it is difficult to comprehensively improve skills related to activity.

### Summary of Invention

The invention discloses a computer-implemented method as set out by claim 1.

The invention discloses a computer-readable recording medium as set out by claim 12.

The invention discloses an information processing system as set out by claim 13.

The invention is defined by the appended claims.

### Brief Description of Drawings

Fig. 1 is a diagram illustrating an activity support system.
Fig. 2 is a block diagram illustrating a functional configuration of a server.
Fig. 3 is a block diagram illustrating a functional configuration of a terminal device.
Fig. 4 is a block diagram illustrating a functional configuration of a wearable device.
Fig. 5 is a diagram illustrating a content example of event list data.
Fig. 6 is a diagram illustrating a content example of event management data.
Fig. 7 is a diagram illustrating a content example of ranking data.
Fig. 8 is a flowchart illustrating a control procedure for event training execution processing.
Fig. 9 is a flowchart illustrating a control procedure for event execution processing by the CPU of the server.
Fig. 10 is a flowchart illustrating a control procedure for event execution processing by the CPU of the terminal device.
Fig. 11 is a flowchart illustrating a control procedure for event execution processing by the CPU of the wearable device.
Fig. 12 is a diagram illustrating a content example of event list data according to Modification 1.
Fig. 13 is a flowchart illustrating a control procedure for event training execution processing according to Modification 1.
Fig. 14 is a diagram illustrating a content example of event list data according to Modification 2.
Fig. 15 is a diagram illustrating a content example of event list data according to Modification 3.
Fig. 16 is a diagram illustrating a content example of event list data according to Modification 4.
Fig. 17 is a diagram illustrating a content example of event list data according to Modification 5.
Fig. 18 is a flowchart illustrating a control procedure for event training execution processing according to Modification 6.

### Description of Embodiment

Hereinafter, an embodiment of the present invention will be described with reference to the drawings.

### <Outline of configuration and operation of activity support System>

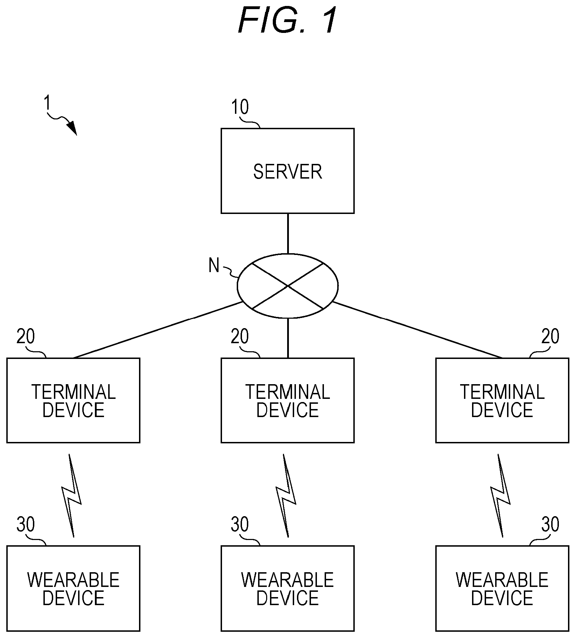

Fig. 1 is a diagram illustrating an activity support system 1 of the present embodiment.

The activity support system 1 (information processing system) includes a server 10 (information processing device), a plurality of terminal devices 20, and a plurality of wearable devices 30 (sensors). The plurality of terminal devices 20 are connected to the server 10 via a network N and can transmit and receive data to and from the server 10. The network N is, for example, the Internet, but is not limited thereto. The communication path between the terminal device 20 and the server 10 may include a wireless communication path.

A user (subject) of the activity support system 1 uses at least one terminal device 20 and at least one wearable device 30. The activity support system 1 provides the user with event training made up of a plurality of events. **In** the event training, it is possible to efficiently and comprehensively improve a skill related to activity by performing activity designated in each of a plurality of events. The activity support system 1 evaluates the activity state of the user in each of the plurality of events that make up event training. Specifically, in each event, the wearable device 30 worn by the user on the body acquires an activity index indicating a performance status such as a moving speed and a moving distance of the user performing the activity and the movement of the body, the activity index is transmitted to the server 10 via the terminal device 20, and the evaluation of the activity state of the user is derived based on the activity index in the server 10. The evaluation result of the activity state is transmitted from the server 10 to the terminal device 20 and presented to the user. **In** this manner, each event making up the event training is executed online (in the present embodiment, via the network N).

**In** a first event (first event) of the plurality of events that make up the event training, a first activity state of the user when the user is performing a first activity is evaluated, and in at least one event (second event) executed after the first event, a second activity state, which is the activity state of the user when the user is performing a second activity and which is different from the first activity state, is evaluated. The first activity and the second activity are, for example, walking or running. In the present embodiment, a case where both the first activity and the second activity are running will be described as an example. Note that the first activity and the second activity may be different from each other. The activity state to be evaluated when the activity is walking or running includes, for example, at least one of items related to the left-right symmetry of the activity of the user, the posture of the user, and the ground contact state of the user. Here, the ground contact means that the user's foot (sole) makes contact with the ground.

The activity support system 1 of the present embodiment includes a plurality of terminal devices 20 and a plurality of wearable devices 30 used by a plurality of different users. Two or more users (hereinafter referred to as "participating users") who are at least some of the plurality of users participate in each of the plurality of events that make up the event training, and the activity states of the participating users are relatively evaluated in each event. In the present embodiment, in each event, the ranking of the participating users is generated based on the evaluation values of the activity states of the participating users, and the rank in the ranking is presented to each user as the evaluation result of the activity state. Specifically, in a certain event (the first event) of the plurality of events, the first activity state of each of the participating users performing the first activity is relatively evaluated and the rank is presented, and in at least one event (the second event) different from the certain event, the second activity state of each of the participating users performing the second activity is relatively evaluated and the rank is presented. In each of the plurality of events that make up the event training, the user can efficiently and comprehensively improve the skill related to running by performing running such that the rank becomes high (such that the evaluation value of the activity state serving as the evaluation index becomes high). The user can also perform two or more times of running in each event and check the rank for each event. This makes it possible to improve the way of running of the user with reference to the change in the rank.

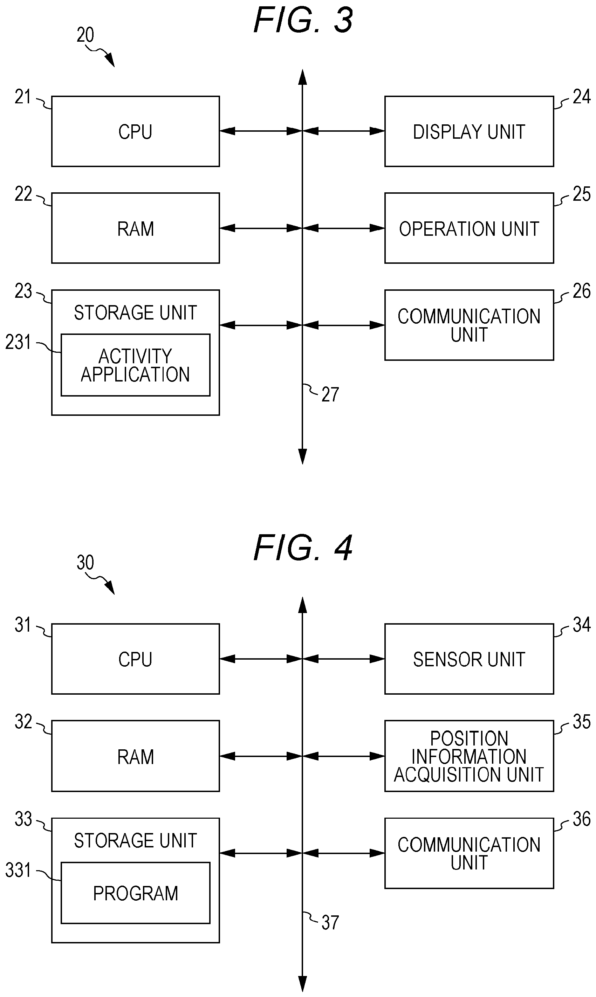

The wearable device 30 is a wearable terminal used by being worn on the user's body (e.g., waist). The wearable device 30 includes a sensor unit 34 (cf. Fig. 4) that detects the movement of the wearable device itself and derives index values of various activity indexes related to the movement of the body of the user wearing the wearable device 30 based on the detection result of the sensor unit 34. The wearable device 30 includes a position information acquisition unit 35 (cf. Fig. 4) that acquires the position information of the wearable device itself, and derives a moving distance, a moving speed, and the like of the user wearing the wearable device 30 based on the position information acquired by the position information acquisition unit 35. The wearable device 30 can transmit and receive data to and from the terminal device 20 by wireless communication (e.g., short-range wireless communications such as Bluetooth (registered trademark)) and transmits data related to an activity index, a moving distance, a moving speed, and the like of the user to the terminal device 20.

The terminal device 20 is a device mainly carried and used by the user and is, for example, a smartphone. In the terminal device 20, an application program (hereinafter referred to as an "activity application 231" (cf. Fig. 3)) is installed to provide the user with a service for supporting the activity of the user. In a state where the activity application 231 is being executed (i.e., on the activity application 231), the terminal device 20 receives the data of the activity index related to the activity from the wearable device 30 and displays the information related to the activity index and the information related to the activity state derived from the activity index on the display unit 24 (cf. Fig. 3). Further, the terminal device 20 transmits the received data of the activity index to the server 10, receives the data of the evaluation result (e.g., the rank in the ranking described above) of the activity state by the server 10, and displays the data on the display unit 24.

Note that the terminal device 20 is not limited to a smartphone but may be a device used by being worn by the user on the body, such as a smartwatch. The terminal device 20 is not limited to a device that can be carried by the user during activity but may be one that transmits and receives information to and from the server 10 and the wearable device 30 after activity. For example, the terminal device 20 may be a tablet terminal, a notebook PC, or the like, or may be a stationary terminal such as a desktop PC.

The server 10 receives and records the data of the activity index related to the activity of the user from the terminal device 20. Further, the server 10 derives the evaluation (e.g., the rank in the ranking described above) of the activity state of the user based on the recorded information and transmits the data of the evaluation result to the terminal device 20. Note that the terminal device 20 may be physically the same as the server 10.

### <Configuration of server>

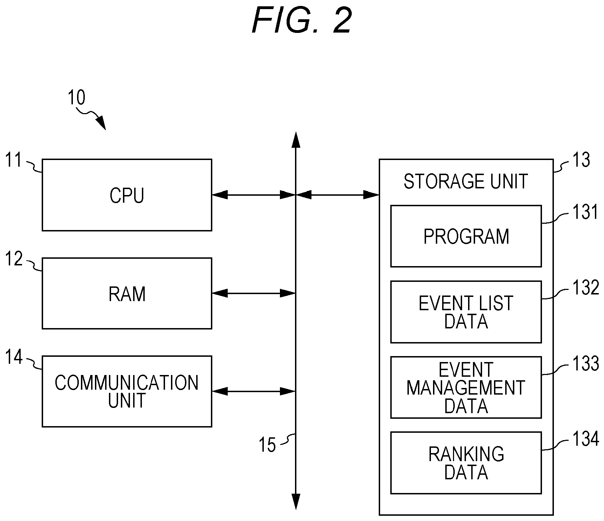

Fig. 2 is a block diagram illustrating the functional configuration of the server 10.

The server 10 includes a central processing unit (CPU) 11, a random access memory (RAM) 12, a storage unit 13, a communication unit 14, a bus 15, and the like. Each unit of the server 10 is connected via the bus 15. Note that the server 10 may further include an operation unit, a display unit, and the like used by an administrator of the server 10.

The CPU 11 is a processor that reads and executes a server a program 131 stored in the storage unit 13 and performs various types of arithmetic processing to control the operation of the server 10. In the present embodiment, the CPU 11 corresponds to a "computer" (or a "processing unit"). Note that the "computer" may include a plurality of processors (e.g., a plurality of CPUs), and the plurality of processors may execute a plurality of type of processing executed by the CPU 11 of the present embodiment. In this case, the plurality of processors correspond to a "computer". In this case, the plurality of processors may be involved in common processing, or each of the plurality of processors may independently execute different processing in parallel.

The RAM 12 provides the CPU 11 with a working memory space and stores temporary data.

The storage unit 13 is a non-transitory recording medium readable by the CPU 11 as a computer and stores the program 131 and various data. The storage unit 13 includes, for example, a non-volatile memory such as flash memory. The program 131 is stored in the storage unit 13 in the form of a computer-readable program code. The storage unit 13 stores event list data 132, event management data 133, ranking data 134, and the like. The event list data 132, the event management data 133, and the ranking data 134 are data used when the event training described above is executed, and specific contents thereof will be described later.

The communication unit 14 performs a communication operation according to a predetermined communication standard. Through this communication operation, the communication unit 14 transmits and receives data to and from the terminal device 20 via the network N.

### <Configuration of terminal device>

Fig. 3 is a block diagram illustrating the functional configuration of the terminal device 20.

The terminal device 20 includes a CPU 21, a RAM 22, a storage unit 23, a display unit 24, an operation unit 25, a communication unit 26, a bus 27, and the like. Each unit of the terminal device 20 is connected via a bus 27.

The CPU 21 is a processor that reads and executes a program such as the activity application 231 stored in the storage unit 23 and performs various arithmetic processing to control the operation of each unit of the terminal device 20. Note that the terminal device 20 may include a plurality of processors (e.g., a plurality of CPUs), and the plurality of processors may execute a plurality of types of processing executed by the CPU 21 of the present embodiment. In this case, the plurality of processors may be involved in common processing, or each of the plurality of processors may independently execute different processing in parallel.

The RAM 22 provides the CPU 21 with a working memory space and stores temporary data.

The storage unit 23 is a non-transitory recording medium readable by the CPU 21 serving as a computer and stores programs such as the activity application 231 and various data. The storage unit 23 includes, for example, a non-volatile memory such as flash memory. The program is stored in the storage unit 23 in the form of a computer-readable program code.

Under the control of the CPU 21, the display unit 24 displays the operation screen of the activity application 231 and various types of information such as the execution status of the event and the rank in the ranking described above. As the display unit 24, for example, a liquid crystal display device that performs display in a dot matrix system can be used, but the present invention is not limited thereto.

The operation unit 25 receives the input operation of the user and outputs an input signal corresponding to the input operation to the CPU 21. The operation unit 25 includes a touch panel provided to be superimposed on the display screen of the display unit 24, and the touch panel detects the touch of the user's finger or the like as an input operation. The operation unit 25 may include a hardware button together with the touch panel or instead of the touch panel, and may be capable of receiving an input operation with the hardware button.

The communication unit 26 performs a communication operation according to a predetermined communication standard. Through this communication operation, the communication unit 26 transmits and receives data to and from the server 10 via the network N. The communication unit 26 transmits and receives data to and from the wearable device 30 by wireless communication (in the present embodiment, Bluetooth as short-range wireless communications).

### <Configuration of wearable device>

Fig. 4 is a block diagram illustrating the functional configuration of the wearable device 30.

The wearable device 30 includes a CPU 31, a RAM 32, a storage unit 33, a sensor unit 34, a position information acquisition unit 35, a communication unit 36, a bus 37, and the like. Each unit of the wearable device 30 is connected via the bus 37.

The CPU 31 is a processor that reads and executes the program 331 stored in the storage unit 33 and performs various types of arithmetic processing to control the operation of each unit of the wearable device 30. Note that the wearable device 30 may include a plurality of processors (e.g., a plurality of CPUs), and the plurality of processors may execute a plurality of types of processing executed by the CPU 31 of the present embodiment. In this case, the plurality of processors may be involved in common processing, or each of the plurality of processors may independently execute different processing in parallel.

The RAM 32 provides the CPU 31 with a working memory space and stores temporary data.

The storage unit 33 is a non-transitory recording medium readable by the CPU 31 as a computer and stores the program 331 and various data. The storage unit 33 includes, for example, a non-volatile memory such as a flash memory. The program 331 is stored in the storage unit 33 in the form of a computer-readable program code.

The sensor unit 34 includes, for example, a triaxial acceleration sensor, a triaxial gyro sensor, and a triaxial geomagnetic sensor as sensors for detecting the activity state of the wearable device 30. The triaxial acceleration sensor detects acceleration in each axial direction applied to the wearable device 30 in accordance with the activity of the user at a predetermined sampling frequency and outputs acceleration data to the CPU 31 as a detection result. The triaxial gyro sensor detects an angular velocity around each axis applied to the wearable device 30 in accordance with the activity of the user at a predetermined sampling frequency and outputs angular velocity data to the CPU 31 as a detection result. The triaxial geomagnetic sensor detects the magnitude of geomagnetism passing through the wearable device 30 at a predetermined sampling frequency and outputs geomagnetism data to the CPU 31 as a detection result. Data output from each of the triaxial acceleration sensor, the triaxial gyro sensor, and the triaxial geomagnetic sensor includes signal components for three axes orthogonal to each other. The sensor unit 34 includes an amplifier (not illustrated) that amplifies analog signals output from the triaxial acceleration sensor, the triaxial gyro sensor, and the triaxial geomagnetic sensor, and an AD converter (not illustrated) that converts the amplified analog signal into digital data and outputs the digital data to the CPU 31. Note that the sensor unit 34 only needs to be able to detect the activity state of the wearable device 30 and is not limited to the configuration including the triaxial acceleration sensor, the triaxial gyro sensor, and the triaxial geomagnetic sensor.

The position information acquisition unit 35 receives and decodes a transmission radio wave from a positioning satellite of a global navigation satellite system (GNSS) such as a global positioning system (GPS) to calculate the current position. The position information acquisition unit 35 calculates the current position under the control of the CPU 31 and outputs the result to the CPU 31.

Note that the method for calculating the current position by the position information acquisition unit 35 is not limited to the method using the transmission radio wave from the positioning satellite but may be, for example, a method of specifying a positional relationship with a beacon installed at a predetermined position based on a signal from the beacon.

Note that a position information acquisition unit having a function similar to that of the position information acquisition unit 35 may be provided in the terminal device 20, and when the terminal device 20 is being carried and used by the user, the position information acquisition unit of the terminal device 20 may calculate the current position.

The communication unit 36 performs a communication operation according to a predetermined communication standard. Through this communication operation, the communication unit 36 transmits and receives data to and from the terminal device 20 by wireless communication (in the present embodiment, Bluetooth as short-range wireless communications).

In addition to the configuration described above, the wearable device 30 may include, for example, an operation unit for receiving an instruction (reporting) to start and end the activity by the user.

### <Operation of activity support system>

Next, the operation of the activity support system 1 will be described focusing on an operation related to the execution of the event training.

In the activity support system 1, when the user makes a request to participate in the event training on the activity application 231 of the terminal device 20, a plurality of events to be incorporated in the event training are selected, and the event list data 132 is generated.

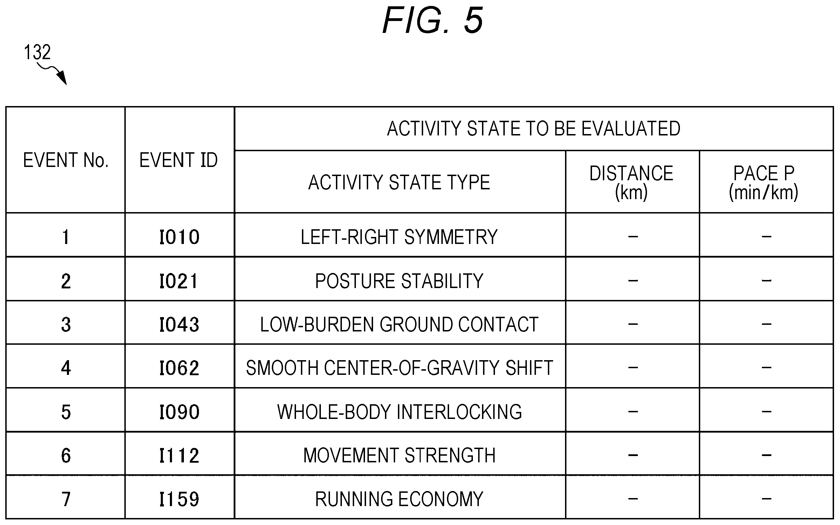

Fig. 5 is a diagram illustrating a content example of the event list data 132.

One data row in the event list data 132 corresponds to one of the events that make up the event training. The event list data 132 has data items of "event No.", "event ID", and "activity state to be evaluated". Among them, the data item of the "activity state to be evaluated" further includes sub-data items of "activity state type", "distance", and "pace P".

The "event No." represents the order in which the events in the data row are executed in the event training. In the event list data 132 illustrated in Fig. 5, the event training is made up of a total of seven events with the "event No." of "1" to "7".

The "event ID" is a unique code allocated to each event and is common to the code in the "event ID" of the event management data 133 to be described later.

The "activity state to be evaluated" represents a condition of the activity state to be evaluated in the event of the data row (hereinafter, the "activity state to be evaluated" is also referred to as an "evaluation condition").

Specifically, the "activity state type" in the "activity state to be evaluated" represents the type of the activity state to be evaluated in the event. In the event list data 132 illustrated in Fig. 5, in the events with the "event No." of "1" to "7", "left-right symmetry", "posture stability", "low-burden ground contact", "smooth center-of-gravity shift", "whole-body interlocking", "movement strength", and "running economy" have been set as the activity states, respectively.

The "distance" in the "activity state to be evaluated" represents the setting of the distance of running to be evaluated. In the example illustrated in Fig. 5, the distance has not been set in each event, and any distance may be set. In a case where a distance has been set in the sub-data item of the "distance", the activity state when running the set distance is performed is to be evaluated, and the activity state when the running distance is less than the set distance is not to be evaluated.

The "pace P" in the "activity state to be evaluated" represents the setting of the range of the pace of running to be evaluated. In the example illustrated in Fig. 5, no pace has been set in each event, and any pace may be set. When a pace has been set in the sub-data item of the "pace P", the activity state in running with the average pace within the designated range is to be evaluated, and the activity state in running with the average pace not within the designated range is not to be evaluated. Here, the average pace is a value obtained by dividing the elapsed time (min) from the start to the end of running by the moving distance (km). Needless to say, the pace can be substituted by a speed.

In the event list data 132, a plurality of events are selected such that the activity state to be evaluated in the first event (a first event) among the plurality of events that make up the event training (a first activity state; the "left-right symmetry" in the example of Fig. 5) is different from the activity state to be evaluated in at least one other event (a second event) performed after the first event (a second activity state; e.g., the "posture stability" when the second event in Fig. 5 is the "second event").

Here, that the activity states to be evaluated in two events are different from each other means that combinations of the sub-data items of the "activity state type", "distance", and "pace P" in the event list data 132 are different from each other. Therefore, in two events, even when the contents of the "activity state type" are the same, in a case where the contents of at least one of the "distance" and the "pace P" are different from each other, the activity states to be evaluated shall be different from each other.

Here, the activity state to be evaluated and the activity index used for evaluating the activity state will be described.

The activity state to be evaluated in the present embodiment includes "left-right symmetry", "posture stability", "low-burden ground contact", "smooth center-of-gravity shift", "whole-body interlocking", "movement strength", and "running economy". Among them, the "left-right symmetry" corresponds to the "left-right symmetry of the activity of the subject", the "posture stability", "smooth center-of-gravity shift", and "movement strength" correspond to the "subject posture", the "low-burden ground contact" corresponds to the "ground contact state of the subject", and "running economy" corresponds to the "left-right symmetry of the activity of the subject", "subject posture", and "ground contact state of the subject".

Examples of the activity indexes used for the evaluation of these activity states include a "pitch", "stride", "stride height ratio", "ground contact time", "ground contact time rate", "initial ground contact position", "landing impact", "ankle turning direction", "ankle turning angle", "ankle turning angular velocity", "knee turning direction", "knee turning angle", "knee turning angular velocity", "vertical movement", "vertical movement height ratio", "sinking", "left-right movement", "left-right-direction impact", "front-back movement", "impulse", "propulsion magnitude", "propulsion direction", "propulsion timing", "brake magnitude", "brake direction", "swing leg swing-backward time", "swing leg swing-forward time", and "placement time", "trunk backward tilt", "trunk forward tilt", "pelvis left-right tilt", "pelvis lift", "pelvis rotation", "pelvis rotation angle in each axis", "pelvis rotation timing", "kicking time", "kicking acceleration", "deceleration amount", "stiffness", "arm swing magnitude", "arm swing direction", "arm swing timing", "head tilt", "head left-right movement", and "head front-back movement".

Each of the activity states to be evaluated will be described below.

The "left-right symmetry" represents whether there is no difference in feature between the left and right of the body and the user is being able to run in a laterally well-balanced manner, and is one of the activity states indicating the burden on the user's body. The "left-right symmetry" is derived by a known predetermined algorithm based on the activity state of, for example, at least one or more of items calculated mainly for each of the left-right legs and each of the left and right arms, such as the "pitch", "stride", "stiffness", "left-right-direction impact", and "left-right movement", among the activity indexes.

The "posture stability" represents whether there is not an unstable movement of the pelvis, and is one of the activity states representing the burden on the user's body (muscles and joints). The "posture stability" is derived by a known predetermined algorithm based on, for example, at least one of the "trunk backward tilt", "pelvis left-right tilt", and "left-right-direction impact" among the activity indexes.

The "low-burden ground contact" represents whether the force applied to each of the leg and the waist is kept small in order to reduce the burden due to the impact caused by the contact of the foot with the ground, and is one of the activity states indicating the burden on the user's body. The "low-burden ground contact" is derived by a predetermined algorithm based on, for example, at least one of the "landing impact", "initial ground contact position", "vertical movement", "sinking", "kicking acceleration", "ground contact time", "ground contact time rate", and "placement time" among the activity indexes.

The "smooth center-of-gravity shift" represents whether the left-right shake and the brake are held small in order to move forward efficiently, and is one of the activity states related to the efficient shift of the center of gravity. The "smooth center-of-gravity shift" is derived by a known predetermined algorithm based on, for example, at least one of the "deceleration amount", "left-right-direction impact", "placement time", "left-right movement", "brake magnitude", and "brake direction" among the activity indexes.

The "whole-body interlocking" represents whether a movement for using each part of the body in a balanced manner is possible, and is one of activity states indicating an important movement leading to improvement in performance. The "whole-body interlocking" is derived by a known predetermined algorithm based on, for example, at least one of the "sinking", "pelvis rotation timing", "kicking time", "swing leg swing-backward time", "swing leg swing-forward time", and "placement time" among the activity indexes.

The "movement strength" represents whether a movement for extending the stride and performing strong running is possible, and is one of the activity states indicating an important movement leading to improvement in performance. The "movement strength" is derived by a predetermined algorithm based on, for example, at least one of the "stride height ratio", "pelvis lift", "pelvis rotation (pelvis rotation angle in each axis)", "swing leg swing-backward time", and "swing leg swing-forward time" among the activity indexes.

The "running economy" represents how a running speed can be acquired without unnecessary movement, and is one of important activity states leading to improvement in performance and reduction in load on the body. The "running economy" is derived by a known predetermined algorithm based on, for example, at least one of the "pitch", "stride height ratio", "vertical movement height ratio", "brake magnitude", and "impulse" among the activity indexes.

The evaluation value of the activity state to be evaluated is represented by, for example, a score within a score range from the lowest point to the highest point (e.g., from 0 to 100 points).

As the activity state to be evaluated, a "overall score" obtained by integrating the above activity states may be used. The "overall score" may be simply the average of the scores of the activity states described above or may be derived by a method of adding the scores of the respective activity states with a predetermined weighting, or the like.

Note that the above are examples of the activity state to be evaluated, and the present invention is not limited thereto.

Each of the activity indexes used for evaluating the activity state will be described below. The values of these activity indexes are derived by processing detection results by the sensor unit 34 of the wearable device 30 by various known processing methods.

The "pitch" is the number of steps per minute. At the same pace, a larger numerical value indicates that the user is running in a quicker rhythm, which means that the feet are turning faster.

The "stride" is a distance moved forward in one step from contact with the ground to the next contact. The larger the numerical value, the larger the distance moved forward per step. The "stride" is generally a good activity index when the running speed is important, but it is also known that too big a stride increases an impact and brake during the ground contact phase, which causes a large burden on the body. Thus, the "stride" is an index with different target values and optimum ranges depending on the purpose and skill of running.

The "stride height ratio" is a numerical value indicating the stride by the height ratio. Using the height ratio has the advantage of reducing the effect of the leg length on the stride size and facilitating comparisons with others.

The "ground contact time" is a time from when the foot makes contact with the ground until the foot kicks and leaves the ground.

The "ground contact time rate" is a value indicating the ground contact time as a proportion in one cycle (a time from contact of a certain foot with the ground to the next contact).

The "initial ground contact position" represents which part of the sole of the foot made the initial contact when making contact with the ground, and is roughly divided into the toe, the heel, and the entire sole, with differences in the degree of absorption of impact, the burden on muscles, tendons, and joints, and the utilization of ground reaction force.

The "landing impact" is the magnitude of an impact applied to the body immediately after contact with the ground. In general, the larger the numerical value, the larger the burden on the legs, knees, and body. The "landing impact" is an activity index that is preferably small.

The "ankle turning direction", "ankle turning angle", and "ankle turning angular velocity" indicate, respectively, the direction, the angle of the turning movement, and the angular velocity in the turning movement of entering from the thumb side or the little toe side of the foot with respect to the moving-forward direction in the period from leaving of the ground to contact with the ground, and the excessive turning movement is considered to have a large burden on the joint.

The "knee turning direction", "knee turning angle", and "knee turning angular velocity" represent the direction, angle, and angular velocity of the turning movement of the knee, respectively.

The "vertical movement" represents the magnitude of the difference between the highest point and the lowest point of the body's center of gravity or the waist position in one cycle. The larger the numerical value, the larger the sinking and bouncing of the body in running. In general, the "vertical movement" is an activity index, a smaller value of which is considered to indicate that the movement of the body's center of gravity is efficient. However, the value is small even when the "sinking" is excessively large, and the contribution to the stride during an aerial phase cannot be expected, making it difficult to increase the running speed.

The "vertical movement body height ratio" is a numerical value indicating vertical movement by a body height ratio. Using the height ratio has the advantage of reducing the effect of the leg length on the body size and facilitating comparisons with others.

The "sinking" represents the range of variation in height from the ground contact time point to a time point at which the body's center of gravity or waist position is at its lowest with the knees mainly flexed. The larger the numerical value, the deeper the sinking, and the sinking may be expressed as a ratio with height as 100%. When the body sinks deeper than necessary during the ground contact phase, a movement of lifting the body's gravity of weight by the amount of the sinking is required during the leg-swinging phase, which is a movement not contributing to the propulsion. Thus, the "sinking" is generally is an activity index that is preferably small. On the other hand, when the sinking is excessively small, the burden on the body cannot be absorbed by each joint because the pelvis, knees, or ankles are not flexed, and hence the burden on the body increases.

The "left-right movement" represents the movement degree in the left-right direction of the body's center of gravity or waist position in one cycle.

The "left-right-direction impact" represents the magnitude of an impact applied in the left-right direction of the body. In general, the larger the numerical value, the more unstable the running in the left-right direction. The "left-right-direction impact" is an activity index that is preferably small.

The "front-back movement" represents the movement degree in the front-back direction of the body's center of gravity or waist position in one cycle.

The "impulse" is an index of the dynamic efficiency of running, obtained by integrating detected value of the acceleration sensor of the sensor unit 34 over one cycle, for example, when the same running speed is maintained between individuals, a smaller impulse indicates that a running speed due to a more efficient body movement has been able to be obtained.

The "propulsion magnitude" is an index of the forward propulsive force, obtained by integrating the forward component of the detected value of the acceleration sensor in the ground contact period over one cycle.

The "propulsion direction" is a direction in which a propulsive force from kicking is directed, and is, for example, the direction of the average of acceleration vectors during a period when an acceleration has a propulsion direction component in the ground contact period.

The "propulsion timing" is a timing at which the acceleration switches from the brake direction to the propulsion direction after contact with the ground.

The "brake magnitude" is an index of the magnitude of the rearward brake force, obtained by integrating the rearward component of the detected value of the acceleration sensor in the ground contact period over one cycle.

The "brake direction" is a direction in which the brake force in the ground contact period is directed, and is, for example, the direction of the average of acceleration vectors during a period when an acceleration has a brake direction component in the ground contact period.

The "swing-backward time of the swing leg" represents the length of a period in which the leg (swing leg) in the leg-swinging phase (a period in which the foot is not in contact with the ground) is swung backward.

The "swing leg swing-forward time" represents the length of a period in which the leg in the leg-swinging phase is swung forward.

The "placement time" represents the length of a period from contact with the ground until the center of gravity is placed on the ground contact foot. The "placement time" can be calculated as the time of the period between the ground contact timing and the "propulsion timing" described above (the timing at which the acceleration switches from the brake direction to the propulsion direction).

The "trunk backward tilt" represents the tilting movement of the trunk, with the pelvis at the center, in the opposite direction to the moving-forward direction during contact with the ground, and the larger the numerical value, the more unstable, the posture of the trunk. The "trunk backward tilt" is an activity index that is considered to be preferably small.

The "trunk forward tilt" represents the tilting movement of the trunk, with the pelvis at the center, in the forward direction with respect to the moving-forward direction during contact with the ground, and is an activity index correlated with the fact that the shift of the body's center of gravity during the ground contact phase is smoothly performed when the value is an appropriate value.

The "pelvis left-right tilt" represents a movement in which the pelvis on the opposite side to the foot in contact with the ground tilts and is lowered at the time of contact with the ground. The larger the numerical value, the more unstable the posture of the pelvis during contact with the ground. The "pelvis left-right tilt" is an activity index that is preferably small.

The "pelvis lift" represents a movement of pulling back the pelvis that has lowered to the swing leg side from contact with the ground to kicking.

The "pelvis rotation" represents a movement of rotating the pelvis. The larger the numerical value, the stronger the running by greatly rotating the pelvis. The "pelvis rotation" is an activity index that is preferably large.

The "pelvis rotation angle in each axis" represents respective rotation angles corresponding to the yaw, roll, and pitching of the pelvis.

The "pelvis rotation timing" represents a gap between the moment of contact with the ground and the timing of rotating the pelvis.

The "kicking time" is a time from when the body sinks to when the foot leaves the ground after contact with the ground. The larger the numerical value, the longer the time required for kicking for each step. The "kicking time" is an activity index that is preferably small.

The "kicking acceleration" is the magnitude of acceleration to kick the ground. In general, the larger the numerical value, the stronger the force with which the player is kicking the ground. The "kicking acceleration" is an activity index that is preferably small under the condition that the same running speed is maintained.

The "deceleration amount" represents the brake magnitude after contact with the ground. In general, the larger the numerical value, the larger the brake for each step. The "deceleration amount" is an activity index that is preferably small.

The "stiffness" represents the hardness of a "spring" when the entire leg is regarded as the spring. In general, the larger the numerical value, the smaller the sinking after contact with the ground, indicating that running is being performed like a "hard spring" that kicks the ground in a short ground contact time.

The "arm swing magnitude" represents the magnitude of the rotation angle range of the arm around the shoulder in one cycle of walking or running movement.

The "arm swing direction" is represented by an angle formed with respect to the moving-forward direction when the arm is swung from the front to the back or from the back to the front in one cycle of walking or running movement.

The "arm swing timing" represents whether a shift in temporal timing at which the foot makes contact with the ground based on a time at which the arm is switched from the front to the back or from the back to the front in one cycle of the walking or running movement.

The "head tilt" represents the magnitude of the deviation of the average direction of the head axis (e.g., an axis passing through the neck and the top of the head) from the vertical direction in one cycle of walking or running movement.

The "head left-right movement" represents the movement degree of the head position in the left-right direction in one cycle.

The "head front-back movement" represents the movement degree of the head position in the front-back direction in one cycle.

Note that the above is an example of the activity index. Even when the index names are different, the definitions are often the same in reality, or even when the index names are the same, the definitions are often different, and the invention is not limited thereto. Any activity index itself may be set as the activity state to be evaluated.

The events registered in the event list data 132 of Fig. 5 (the events incorporated in the event training) are selected from all the events managed by the activity support system 1 in the event management data 133.

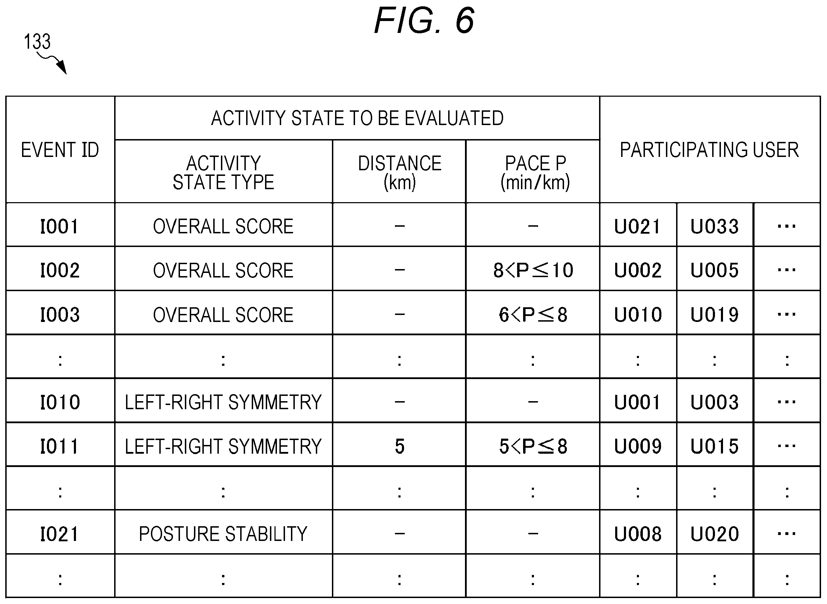

Fig. 6 is a diagram illustrating a content example of the event management data 133.

In the event management data 133, information related to each of a plurality of events being executed in the activity support system 1 is recorded. The event management data 133 may include information related to an event that was executed in the past and has not been executed currently or an event that has not been executed yet. One data row in the event management data 133 corresponds to one event. The event management data 133 has data items of "event ID", "activity state to be evaluated", and "participating user". Among them, the data item of the "activity state to be evaluated" further includes sub-data items of "activity state type", "distance", and "pace".

The "event ID" is a unique code allocated to each event.

The contents represented by the respective sub-data items of the "activity state type", "distance", and "pace" included in the "activity state to be evaluated" are the same as those of the sub-data item in the event list data 132 of Fig. 5.

The "participating user" represents the user ID of the user registered (participating) in the event of the data row. In the present embodiment, not all the participating users are necessarily registered all at once in each event, and not all the participating users are deregistered all at once. In accordance with the event list data 132, a user who is to participate in a certain event participates in the certain event at that time, and is deregistered at the time when the evaluation of the activity state in the event for the user is completed. That is, the time point of participation in the event and the time point of the end of the event are usually different for each user. Therefore, the number of participating users for the event is not necessarily constant and may change at any time.

However, all the participating users may be registered simultaneously in a certain event, or the registration of all the participating users may be canceled simultaneously. **In** other words, the time point of participation in the event and the end time point of the event may be the same for all the participating users of the certain event. All the participating users may participate in all the events of the same event training.

Each event in the event list data 132 illustrated in Fig. 5 is selected from the event management data 133 illustrated in Fig. 6 according to a predetermined rule. In the example illustrated in Fig. 5, the activity states to be evaluated in the plurality of events are selected in the order of "1. Activity state representing burden on user's body" ("left-right symmetry", "posture stability", "low-burden ground contact"), "2. Activity state related to efficient center-of-gravity shift" ("smooth center-of-gravity shift"), and "3. Activity state representing important movement leading to performance improvement" ("whole-body interlocking", "movement strength", "running economy"). In particular, the activity state to be evaluated in the first event (first event) in the event list data 132 is the activity state representing the burden on the user's body due to the activity. With the "running economy" being an activity state that also leads to a reduction in load on the body, the "running economy" may be included in "1. Activity state representing burden on user's body". That is, the event in which the "running economy" is set as the activity state to be evaluated may be executed before the event in which the "smooth center-of-gravity shift" is set as the evaluation state.

By performing the events in this order, the skill for reducing the burden on the body can be improved with priority, enabling the remaining events to be performed with less burden on the body and less chance of injury. As a result, the event period can be lengthened, effectively improving the skill related to activity.

When the event list data 132 illustrated in Fig. 5 is generated, events (here, seven events) are sequentially executed in the order illustrated in the "event No." of the event list data 132. In each event, the activity state to be evaluated (evaluation condition) for the event is presented to the user via the terminal device 20, and the user performs an activity that meets the evaluation condition while wearing the wearable device 30. The activity index acquired by the wearable device 30 during activity is transmitted to the server 10 via the terminal device 20. When the activity ends, the evaluation value (score) of the activity state of the user is derived in the server 10. Further, the ranking data 134 of the participating users is generated based on the evaluation values of all the participating users of the event.

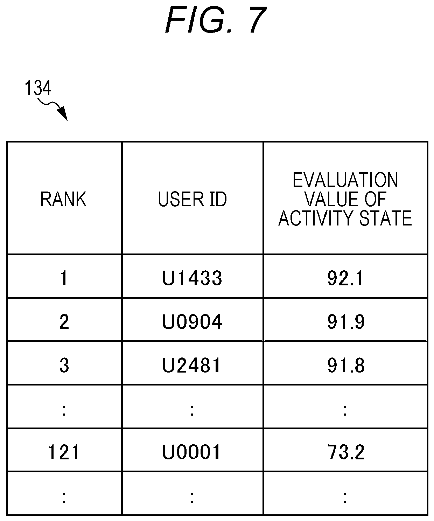

Fig. 7 is a diagram illustrating a content example of the ranking data 134.

In the ranking data 134, information of ranking based on the evaluation values of the activity states of the participating users in the corresponding event is recorded. The ranking data 134 is generated for each event managed in the event management data 133. The ranking data 134 is updated, for example, at the timing when the evaluation value of any participating user for the corresponding event is generated or updated. The ranking data 134 includes data items of "rank", "user ID", and "evaluation value of activity state".

The "rank" represents the rank of the user of the data row among the participating users.

The "user ID" is a unique code allocated to each user, and the same code as that used for the "participating user" in the event management data 133 of Fig. 6 is used.

The "evaluation value of activity state" represents the evaluation value of the activity state to be evaluated, derived based on the activity index in the activity performed by the user of the data row. The ranking data 134 is obtained by sorting the participating users such that the evaluation values are in descending order.

When the ranking data 134 is generated, the rank in the ranking is presented to the user via the terminal device 20. For example, for a user with a user ID of "U0001" in Fig. 7, it is presented that the rank is "121st". The rank is presented, for example, by at least one of screen display on the display unit 24 of the terminal device 20 and output of a voice by a voice output unit (not illustrated) provided in the terminal device 20.

In each event, when a certain user receives the evaluation of the activity state at least once (i.e., when the rank in the ranking is derived), an event terminable condition of the event is satisfied, and the user can proceed to the next event in the event list of the event training. Note that the event terminable condition may include an additional condition other than receiving the evaluation of the activity state at least once. Examples of the additional condition include that a predetermined period has elapsed from the start of the event and that the evaluation of the activity state has become equal to or higher than a predetermined standard (that the rank in the ranking has become equal to or higher than a certain reference rank). The additional condition included in the event terminable condition may be separately determined for each event or for each user.

Alternatively, the user may determine whether or not to proceed to the next event when the event terminable condition is satisfied. When the process does not proceed to the next event, it may be possible to perform the activity according to the evaluation condition again (hereinafter referred to as "retry" of activity) and receive the evaluation of the activity state. Every time the retry of the activity is performed, a rank in accordance with the latest activity state of the activity is derived and presented to the user.

When all the events included in the event list data 132 end, the event training ends.

### <Processing related to execution of event training>

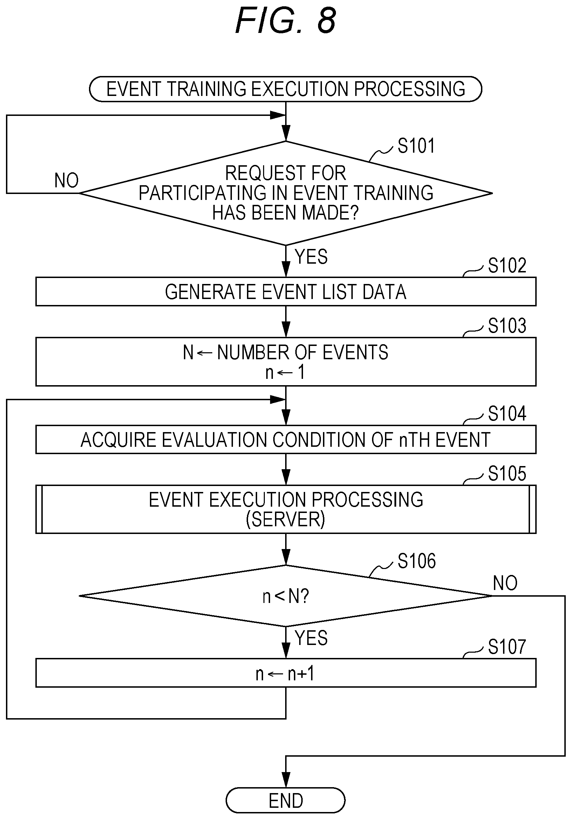

Next, control procedures for processing performed by the CPU 11 of the server 10, the CPU 21 of the terminal device 20, and the CPU 31 of the wearable device 30 in order to execute the event training described above will be described with reference to flowcharts of Figs. 8 to 11.

Fig. 8 is a flowchart illustrating a control procedure for event training execution processing.

The event training execution processing is executed by the CPU 11 of the server 10.

When the event training execution processing is started, the CPU 11 determines whether or not a request to participate in the event training by the user has been transmitted from the terminal device 20 (step S101). The participation request to the event training is transmitted from the terminal device 20 to the server 10, for example, when the user performs a predetermined operation for requesting to participate in the event training on the activity application 231 of the terminal device 20. When determining that the request to participate in the event training has not been transmitted ("NO" in step S101), the CPU 11 executes the processing of step S101 again.

When determining that the request to participate in the event training has been transmitted ("YES" in step S101), the CPU 11 selects an event to be incorporated into the event training from the events managed in the event management data 133 and generates the event list data 132 (step S102). The CPU 11 substitutes the number of events in the generated event list data 132 to a variable N and substitutes "1" to a variable n (step S103).

The CPU 11 refers to the event list data 132 to acquire the evaluation condition (settings of the "activity state type", "distance", and "pace P" in the event list data 132 in Fig. 5) of the nth event (step S104). The CPU 11 executes event execution processing to be described later for the nth event (step S105).

When the event execution processing ends, the CPU 11 determines whether or not the value of the variable n is less than the value of the variable N (step S106). When determining that the value of the variable n is less than the value of the variable N ("YES" in step S106), the CPU 11 substitutes "n + 1" for the variable n (step S107), and returns the processing to step S104 to execute the processing related to the next event.

When determining that the value of the variable n matches the value of the variable N ("NO" in step S106), the CPU 11 ends the event training execution processing.

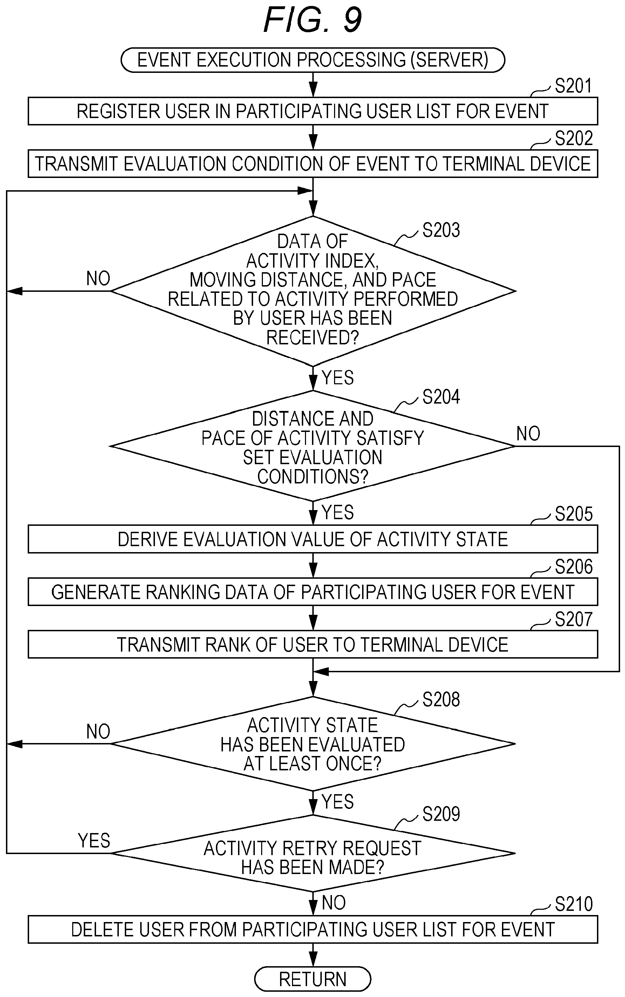

Fig. 9 is a flowchart illustrating a control procedure for event execution processing by the CPU 11 of the server 10.

When the event execution processing is called, the CPU 11 registers the user in the list of participating users for the nth event (step S201). That is, the CPU 11 registers the user ID of the user in the data item of "participating user" in the event management data 133 for the nth event. Further, the CPU 11 transmits data related to the evaluation condition of the nth event acquired in step S104 of Fig. 8 to the terminal device 20 (step S202).

The CPU 11 determines whether or not the activity of the user has ended and data of each of the activity index, the moving distance, and the pace related to the activity has been received from the terminal device 20 (step S203). When the distance has not been set as the evaluation condition in the event being executed, the determination related to the reception of the data of the moving distance can be omitted. When the pace has not been set as the evaluation condition in the event being executed, determination related to the reception of data of the pace can be omitted. When determining that the data of each of the activity index, the moving distance, and the pace has not been received ("NO" in step S203), the CPU 11 executes the processing of step S203 again.

When determining that the data of each of the activity index, the moving distance, and the pace is received ("YES" in step S203), the CPU 11 determines whether or not the distance and the pace of the activity performed by the user satisfy the evaluation conditions set in the event (step S204). When the distance and the pace are optional in the event, the CPU 11 skips step S204 and shifts the processing to step S205.

When determining that the distance and the pace of the activity performed by the user satisfy the evaluation conditions set in the event ("YES" in step S204), the CPU 11 derives the evaluation value of the activity state to be evaluated based on the received data of the activity index (step S205). The CPU 11 acquires the evaluation values of the latest activity states of all the participating users registered in the event in the event management data 133 and sorts the participating users in descending order of the evaluation values to generate the ranking data 134 (step S206). The CPU 11 transmits data indicating the rank of the user in the ranking data 134 to the terminal device 20 (step S207).

When step S207 ends or when it is determined in step S204 that the distance and the pace of the activity performed by the user do not satisfy the evaluation conditions set in the event ("NO" in step S204), the CPU 11 determines whether or not the user has been evaluated for the activity state at least once (whether or not the event terminable condition is satisfied) in the event corresponding to the current n which is being executed (step S208). When the event terminable condition includes an additional condition other than receiving the evaluation of the activity state at least once, the CPU 11 determines whether or not the event terminable condition including these additional conditions is satisfied in step S208. When determining that the user has not received the evaluation of the activity state even once (the event terminable condition is not satisfied) ("NO" in step S208), the CPU 21 returns the processing to step S203.

When determining that the user has received the evaluation of the activity state at least once (the event terminable condition is satisfied) ("YES" in step S208), the CPU 11 determines whether or not an activity retry request has been transmitted from the terminal device 20 (step S209). When determining that the activity retry request has been transmitted ("YES" in step S209), the CPU 11 returns the processing to step S203. When determining that the activity retry request has not been transmitted and a shift is to be made to the next event ("NO" in step S209), the CPU 11 deletes the user from the list of participating users for the nth event (step S210). That is, the CPU 11 deletes the user ID of the user from the data item of "participating user" in the event management data 133 for the nth event.

When the processing of step S210 ends, the CPU 11 ends the event execution processing and shifts the processing to step S106 of the event training execution processing.

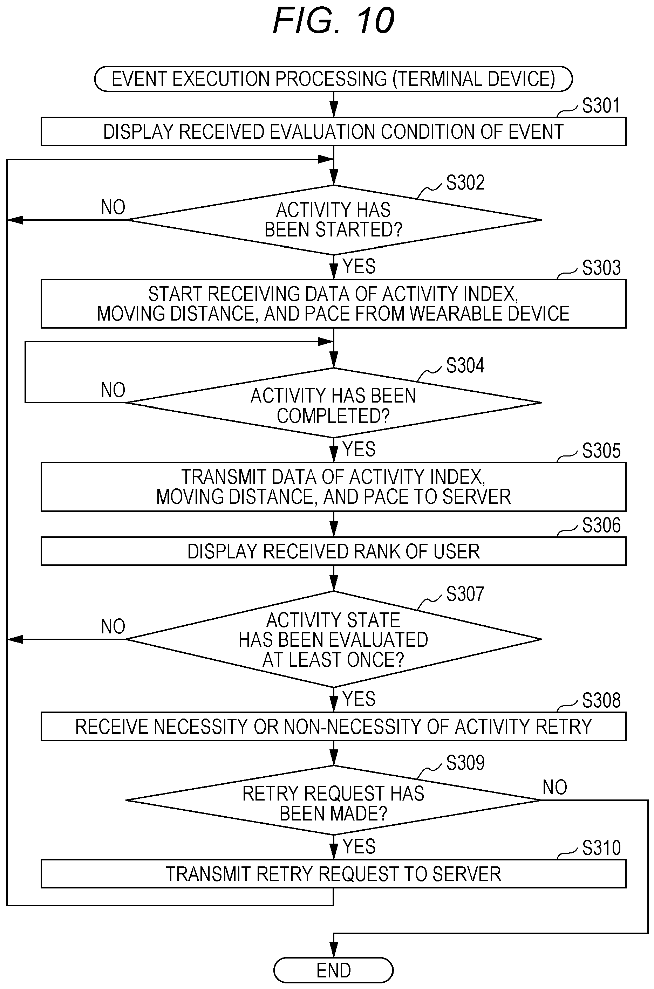

Fig. 10 is a flowchart illustrating a control procedure for event execution processing by the CPU 21 of the terminal device 20.

The event execution processing of Fig. 10 is executed in parallel with the event execution processing by the CPU 11 of the server 10 illustrated in Fig. 9.

When the event execution processing is started, the CPU 21 receives the data related to the evaluation condition of the event transmitted from the server 10 in step S202 of Fig. 9 and causes the display unit 24 to display the evaluation condition of the event (step S301).

The CPU 21 determines whether or not the activity by the user has been started (step S302). For example, the start of the activity may be determined based on the fact that a predetermined operation indicating the start of the activity has been performed by the user on the activity application 231 of the terminal device 20, or may be determined based on reception of a notification from the wearable device 30 that has detected the start of the activity. When determining that the activity has not been started ("NO" in step S302), the CPU 21 executes the processing of step S302 again.

When determining that the activity has been started ("YES" in step S302), the CPU 21 starts receiving the data of each of the activity index, the moving distance, and the pace transmitted from the wearable device 30 (step S303). Instead of the mode of receiving the data of each of the moving distance and the pace from the wearable device 30, a mode may be adopted in which data related to the current position is received from the wearable device 30, and the CPU 21 derives the moving distance and the pace based on the transition of the current position. Thereafter, the CPU 21 sequentially stores and accumulates the data of each of the activity index, the moving distance, and the pace received from the wearable device 30 until the end of the activity in the RAM 22 or the storage unit 23.

The CPU 21 determines whether or not the activity by the user has been completed (step S304). The completion of the activity may be determined, for example, on the activity application 231 of the terminal device 20 based on the fact that a predetermined operation indicating the completion of the activity has been performed by the user, or may be determined based on reception of a notification from the wearable device 30 that has detected the completion of the activity. When determining that the activity has not been completed ("NO" in step S304), the CPU 21 executes the processing of step S304 again while continuing the reception of the data of the activity index.

When determining that the activity has been completed ("YES" in step S304), the CPU 21 transmits the data of each of the activity index, the moving distance, and the pace received from the wearable device 30 for the activity to the server 10 (step S305).

The CPU 21 receives the data indicating the rank of the user in the ranking data 134 transmitted from the server 10 in step S207 of Fig. 9 and causes the display unit 24 to display the rank (step S306).

The CPU 21 determines whether or not the user has received the evaluation of the activity state at least once (whether or not the event terminable condition is satisfied) (step S307). Here, the CPU 21 may uniquely determine whether or not the user has received the evaluation of the activity state at least once (whether or not the evaluation of the activity state satisfies the event terminable condition) or may perform the determination based on whether or not a notification indicating that the user has received the evaluation of the activity state at least once (that the event terminable condition is satisfied) has been received from the server 10. When determining that the user has not received the evaluation of the activity state even once (the event terminable condition is not satisfied) ("NO" in step S307), the CPU 21 returns the processing to step S302.

When determining that the user has received the evaluation of the activity state at least once (the event terminable condition is satisfied) ("YES" in step S307), the CPU 21 receives an operation of selecting necessity or non-necessity of retry of activity by the user (step S308). For example, the CPU 21 causes the display unit 24 to display a dialogue screen inquiring whether or not to retry activity on the activity application 231. The CPU 21 determines whether or not an activity retry request has been made (step S309). When determining that a retry request has been made ("YES" in step S309), a signal requesting retry is transmitted to the server 10 (step S310), and the processing returns to step S302.

When determining that the activity retry request has not been made and a shift is to be made to the next event ("NO" in step S309), the CPU 21 ends the event execution processing.

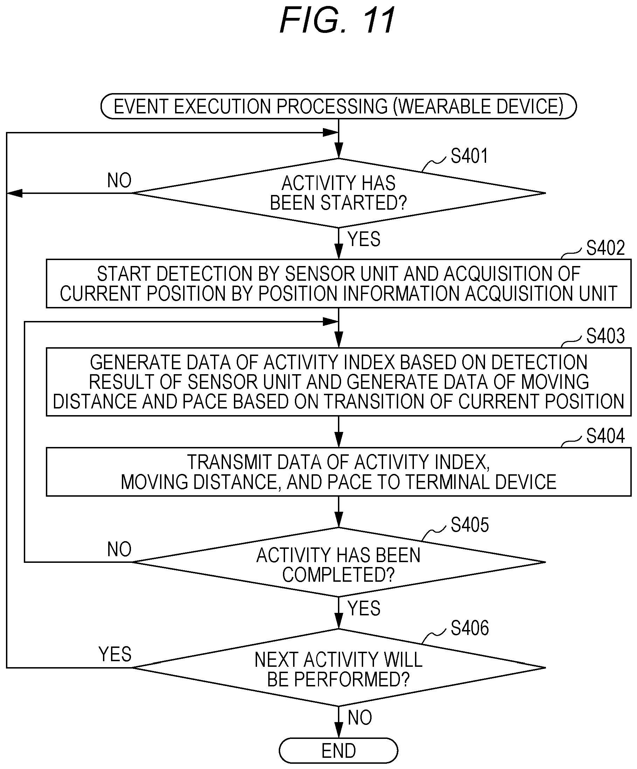

Fig. 11 is a flowchart illustrating a control procedure for event execution processing by the CPU 31 of the wearable device 30.

The event execution processing in Fig. 11 is executed in parallel with the event execution processing by the CPU 21 of the terminal device 20 illustrated in Fig. 10.

When the event execution processing is started, the CPU 31 determines whether or not the activity by the user is started (step S401). For example, the start of the activity may be determined based on the fact that an operation of instructing (reporting) the start of the activity has been performed on an operation unit (not illustrated) provided in the wearable device 30, or may be determined based on reception of a notification from the terminal device 20 that has detected the start of the activity. When determining that the activity has not been started ("NO" in step S401), the CPU 31 executes the processing of step S401 again.

When determining that the activity has been started ("YES" in step S401), the CPU 31 starts detection by the sensor unit 34 and acquisition of the current position by the position information acquisition unit 35 (step S402).

The CPU 31 generates the data of the activity index based on the detection result of the sensor unit 34 at a predetermined timing and generates the data of each of the moving distance and the pace based on the transition of the current position acquired by the position information acquisition unit 35 (step S403). The CPU 31 transmits the generated data of each of the activity index, the moving distance, and the pace to the terminal device 20 (step S404).

The CPU 31 determines whether or not the activity by the user has been completed (step S405). For example, the completion of the activity may be determined based on the fact that an operation of instructing (reporting) the completion of the activity has been performed on the operation unit described above, or may be determined based on reception of a notification from the terminal device 20 that has detected the completion of the activity. When determining that the activity has not been completed ("NO" in step S405), the CPU 31 returns the processing to step S403 and continues the generation and transmission of the data of each of the activity index, the moving distance, and the pace.

When determining that the activity has been completed ("YES" in step S405), the CPU 31 determines whether or not to execute the next activity is to be performed (step S406). For example, when receiving a notification indicating that an activity retry request has been made from the terminal device 20, the CPU 31 determines that the next activity is to be performed. When determining that the next activity is to be performed ("YES" in step S406), the CPU 31 returns the processing to step S401, and when determining that the next activity is not to be performed ("NO" in step S406), the CPU 31 ends the event execution processing.

### <Modifications>

Next, a modification of the above embodiment will be described. In each modification, a difference from the above embodiment will be described, and description of points common to the above embodiment will be omitted.

### (First modification)

The present modification is different from the above embodiment in that an event for evaluating an overall score of activity states is incorporated in the event training.

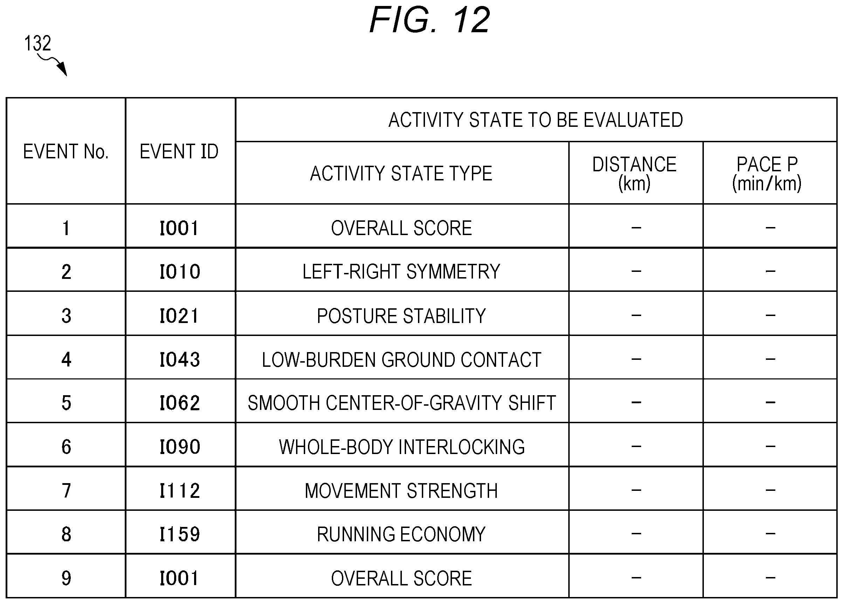

Fig. 12 is a diagram illustrating a content example of the event list data 132 according to Modification 1.

The event list data 132 illustrated in Fig. 12 includes the same events as the event list data 132 illustrated in Fig. 5, and before the first event in Fig. 5 (the event with the "event ID" of "I010"; first event), an event (event ID "1001"; hereinafter referred to as a "comprehensive evaluation event") in which the "activity state type" of the "activity state to be evaluated" is the "overall score" is provided. In the event list data 132 illustrated in Fig. 12, the comprehensive evaluation event is also provided after the last event (the event with the "event ID" of "I159") in the event list data 132 illustrated in Fig. 5. Hereinafter, each of the events except for the comprehensive evaluation event is referred to as a "normal event".

In the comprehensive evaluation event, the activity states to be evaluated in a plurality of normal events included in the event training (in the example illustrated in Fig. 12, "left-right symmetry", "posture stability", "low-burden ground contact", "smooth center-of-gravity shift", "whole-body interlocking", "movement strength", and "running economy") are evaluated comprehensively. The comprehensive evaluation event corresponds to a step of comprehensively evaluating a plurality of different activity states that include the activity state to be evaluated in the first event (first event) among the plurality of normal events and the activity state to be evaluated in any event other than the first event (second event) among the plurality of normal events. Note that the activity states comprehensively evaluated in the comprehensive evaluation event may be the activity states to be evaluated in some events among the plurality of normal events included in the event training. The activity states comprehensively evaluated in the comprehensive evaluation event may include an activity state that is not an evaluation target in the plurality of normal events included in the event training.

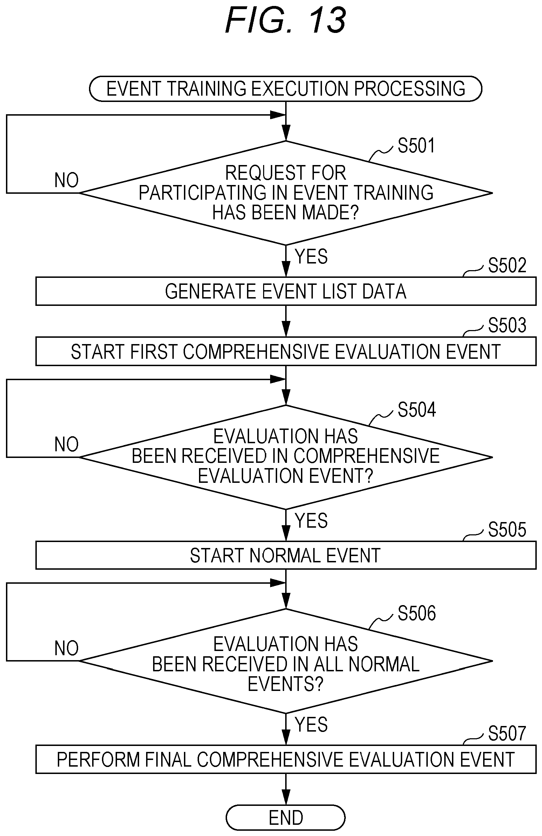

Fig. 13 is a flowchart illustrating a control procedure for event training execution processing according to Modification 1.

The flowchart of Fig. 13 corresponds to a flowchart, with steps S103 to S107 of the event training execution processing of Fig. 8 changed to the contents of the present modification, represented from a viewpoint different from Fig. 8. The processing contents of steps S501 and S502 in Fig. 13 are the same as the processing contents of steps S101 and S102 in Fig. 8, respectively.

When the event list data 132 is generated in step S502, the CPU 11 starts a first comprehensive evaluation event (step S503). The CPU 11 determines whether or not the user has received the evaluation of the activity state in the first comprehensive evaluation event, and when determining that the user has not received the evaluation in the comprehensive evaluation event ("NO" in step S504), the CPU 11 executes the processing of step S504 again. When determining that the user has received an evaluation in the comprehensive evaluation event ("YES" in step S504), the CPU 11 starts a normal event (step S505). By first comprehensively evaluating the activity state in the comprehensive evaluation event as thus described, the user can grasp the comprehensive skill state at the start of the event training.

The CPU 11 determines whether or not the user has received the evaluations of the activity states in all the normal events (including the second event) (step S506), and when determining that the user has not received the evaluation in any of the normal events ("NO" in step S506), the CPU 11 executes the processing of step S506 again. When determining that the user has received evaluations in all the normal events ("YES" in step S506), the CPU 11 executes the last comprehensive evaluation event (step S507). When the processing of step S507 ends, the CPU 11 ends the event training execution processing. By comprehensively evaluating the activity states again in the last comprehensive evaluation event as thus described, the user can grasp how much the comprehensive activity skill of the user has been improved through the event training by comparison with the evaluation in the first comprehensive evaluation event.

### (Modification 2)

The present modification is different from the above embodiment in that, in the event training, events are alternately incorporated such that conditions related to an activity state to be evaluated have a trade-off relationship. The present modification may be combined with the first modification.

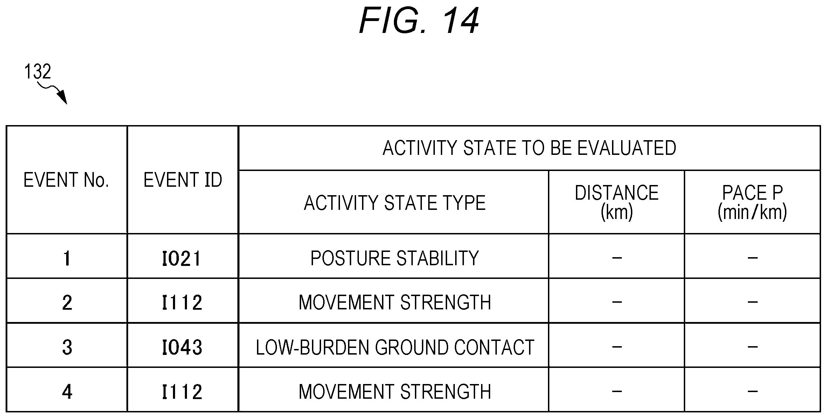

Fig. 14 is a diagram illustrating a content example of the event list data 132 according to Modification 2.

As illustrated in Fig. 14, the event training of the present modification includes four events, and the activity states to be evaluated in these four events are "posture stability", "movement strength", "low-burden ground contact", and "movement strength" in the execution order. In the present modification, the first event corresponds to the first event, and the second event corresponds to the second event.

The "posture stability" of the first event and the "low-burden ground contact" of the third event satisfy a condition (hereinafter referred to as "condition A") of "an activity state related to an important movement for continuing efficient and reasonable running".

The "movement strength" of the second and fourth events satisfies a condition (hereinafter referred to as "condition B") of "an activity state related to an important movement for acquiring a high running speed".

Here, the condition A and the condition B are in a trade-off relationship with each other. Specifically, the activity index satisfying the condition A is an activity index for which the evaluation value of the activity index satisfying the condition B is hardly improved (or easily decreased) due to the movement of the body improving the evaluation value of the activity index, and conversely, the activity index satisfying the condition B is an activity index for which the evaluation value of the activity index satisfying the condition A is hardly improved (or easily decreased) due to the movement of the body improving the evaluation value of the activity index.

Although it is not usually easy to improve the evaluation value of the activity index satisfying the condition A and the evaluation value of the activity index satisfying the condition B at a time, it is possible to efficiently and comprehensively improve the skill related to the activity such that the evaluation values of these activity indexes are naturally improved by alternately incorporating events in which the conditions related to the activity states to be evaluated are in a trade-off relationship as in the present modification.

Note that the conditions related to the activity states that make the trade-off relationship is not limited to the above conditions A and B, and for example, a condition of "an activity state related to an important movement for running a short distance in a shorter time" and a condition of "an activity state related to an important movement for running a long distance in a shorter time" may be used.

### (Modification 3)

The present modification is different from the above embodiment in that the range of the pace of running has been set as an evaluation condition in an event. The present modification may be combined with at least one of Modifications 1 and 2.

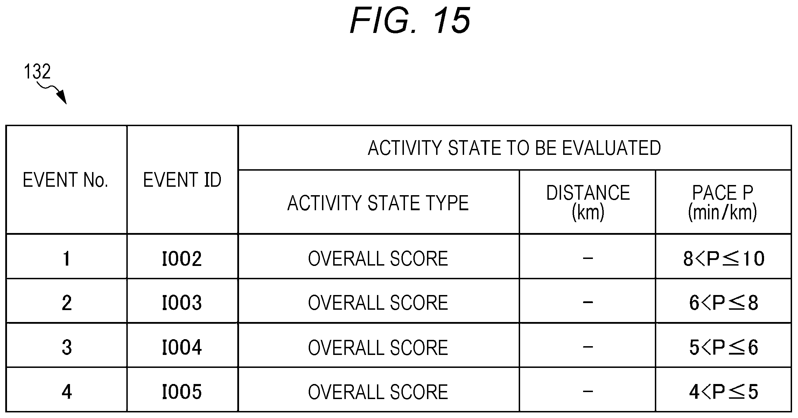

Fig. 15 is a diagram illustrating a content example of the event list data 132 according to Modification 3.

As illustrated in Fig. 15, the event training of the present modification includes four comprehensive evaluation events, in all of which the overall score is an evaluation target. The first event has a condition that the pace P (min/km) satisfies 8 < P ≤ 10, the second event has a condition that the pace P satisfies 6 < P ≤ 8, the third event has a condition that the pace P satisfies 5 < P ≤ 6, and the fourth event has a condition that the pace P satisfies 4 < P ≤ 5. In the present modification, in certain running (activity), only when running is performed with the average pace within the range of the pace set in the evaluation condition, the activity state in the running is evaluated and the rank is derived.

In the example illustrated in Fig. 15, the setting of the pace is gradually faster for each event. Since it is effective to improve the form of running at a slow pace (low speed range), it is possible to maintain the improved form even at a faster pace by first improving the form in the event at a slower pace and gradually performing the event at a faster pace as illustrated in Fig. 15.

Note that at least some of the events incorporated in the event training of the present modification may be set as normal events.

### (Modification 4)

The present modification is different from the above embodiment in that a running distance has been set as an evaluation condition in an event. The present modification may be combined with at least one of Modifications 1 to 3.

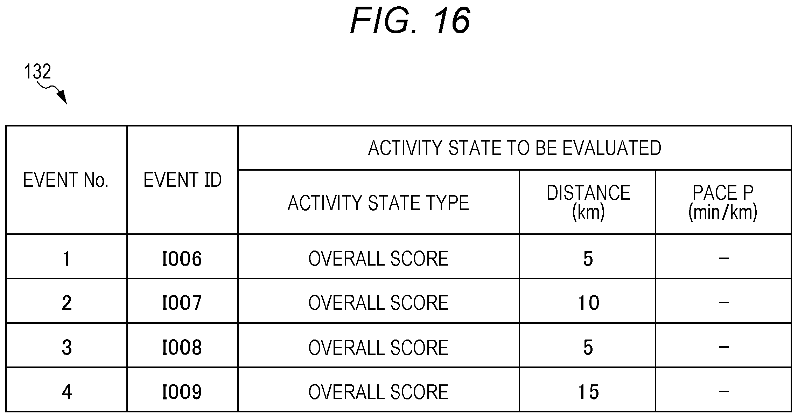

Fig. 16 is a diagram illustrating a content example of the event list data 132 according to Modification 4.

As illustrated in Fig. 16, the event training of the present modification includes four comprehensive evaluation events, in all of which the overall score is an evaluation target. The distance condition has been set to 5 km in the first event km, the distance condition has been set to 10 km in the second event, the distance condition has been set to 5 km in the third event, and the distance condition has been set to 15 km in the fourth event. In the present modification, in certain running (activity), only when running of a distance set in the evaluation condition is performed, the activity state in the running is evaluated, and the rank is derived.

In this manner, in the example illustrated in Fig. 16, the event in which the running distance is set to a short distance (5 km or less) and the event in which the running distance is set to a long distance (10 km or more) are executed alternately. According to this, in each of the short-distance event and the long-distance event, it is possible to find and improve the problem of the form in the speed range corresponding to the distance. Further, by alternately executing the short-distance event and the long-distance event, it is possible to comprehensively improve the form of the short-distance running and the form of the long-distance running. In the example illustrated in Fig. 16, focusing only on the long-distance event, the set distance becomes longer as the event occurs later. By gradually increasing the distance in the long-distance event in this manner, it is possible to naturally improve the form at the time of running a longer distance.

In addition to (or instead of) deriving the rank based on the evaluation value of the activity index in each event, ranking may be generated such that the smaller the difference between the evaluation value of the activity state in one short-distance event and the evaluation value of the activity state in the next long-distance event, the higher the rank, and then the rank may be derived. According to this, it is possible to make the user aware that disturbance of the form due to a change in distance is reduced.

Note that at least some of the events incorporated in the event training of the present modification may be set as normal events.

### (Modification 5)

The present modification is different from the above embodiment in that the inclination of the running path is designated in an evaluation condition in an event. The present modification may be combined with at least one of Modifications 1 to 4.

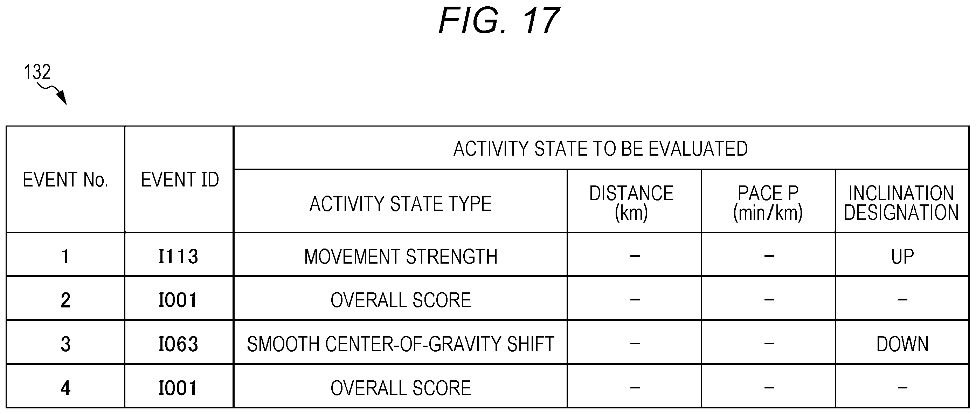

Fig. 17 is a diagram illustrating a content example of the event list data 132 according to Modification 5.

As illustrated in Fig. 17, in the event list data 132 of the present modification, "inclination designation" for designating the inclination (up or down) of the running path is included as the evaluation condition in each event in addition to the "activity state type", "distance", and "pace P". In the example illustrated in Fig. 17, in the first event, the "movement strength" at the time of running on an ascending running path is set as the activity state to be evaluated. In the third event, the "smooth center-of-gravity shift" at the time of running on the downward running path is set as the activity state to be evaluated. In addition, the second and fourth events are comprehensive evaluation events.

The presence or absence of the inclination and the inclination direction (up or down) of the running path, on which the running has been performed, can be determined by providing an altimeter on the wearable device 30 and based on a change in altitude detected by the altimeter. Alternatively, the determination may be made by specifying a running path on the map data from the transition of the current position during running, and a change in altitude along the running path may be derived from the map data.

By designating the inclination of the running path, it is possible to facilitate improvement in the skill related to the specific activity state in accordance with the direction of the inclination. For example, by running on the upward running path, it is possible to facilitate improvement in the skill related to the activity state of "strong movement". By running on the downward running path, it is possible to facilitate improvement in the skill related to the activity state of "smooth center-of-gravity shift". Further, by executing the comprehensive evaluation event next to the ascending or descending event and evaluating the comprehensive activity state, it is possible to confirm the degree of improvement in the skill in the ascending or descending event.

### (Modification 6)

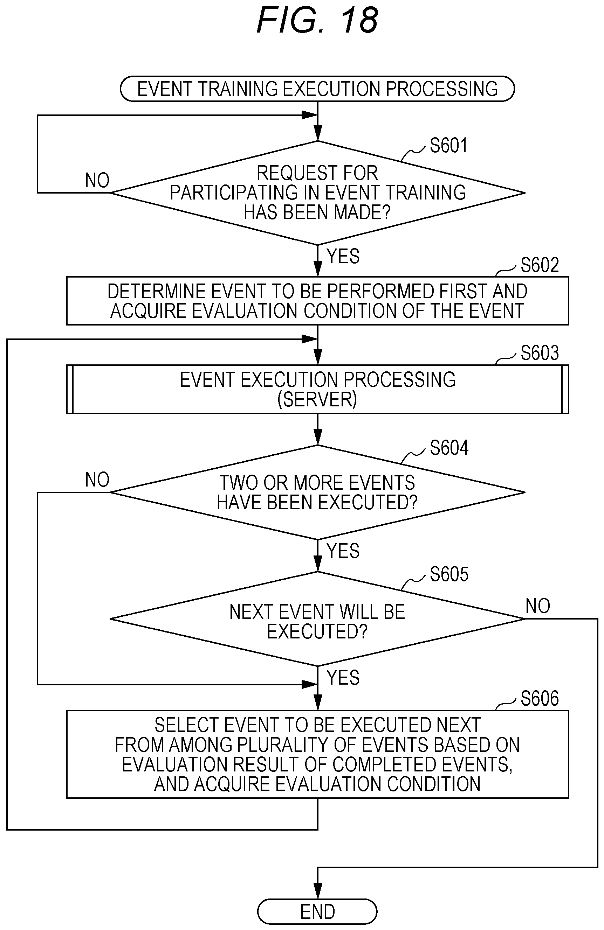

In the above embodiment, when a request to participate in the event training is made, the event list data 132 illustrated in Fig. 5 is generated, and a plurality of events that make up the event training are determined. However, in the present modification, instead of this, contents (evaluation conditions) of the events to be executed thereafter are made different based on a result of evaluation in a certain event of the event training. As a result, the content of the event training can be flexibly changed such that the skill is effectively improved in accordance with the state of the skill related to the activity of the user. The present modification may be combined with at least one of Modifications 1 to 5. Hereinafter, a method for executing the event training in the present modification will be described with reference to the flowchart of Fig. 18.

Fig. 18 is a flowchart illustrating a control procedure for event training execution processing according to Modification 6.

When the event training execution processing is started, the CPU 11 determines whether or not a request to participate in the event training by the user has been transmitted from the terminal device 20 (step S601). The content of the processing in step S601 is the same as that in step S101 in Fig. 8.

When determining that a request to participate in the event training has been transmitted ("YES" in step S601), the CPU 11 determines an event to be performed first from among the events of the event management data 133 according to a predetermined rule and acquires an evaluation condition in the event (step S602). Note that the comprehensive evaluation event may be always executed first.

The CPU 11 executes the event execution processing described above for the determined event (step S603).

The CPU 11 determines whether or not two or more events have been executed after the start of the event training (step S604). When determining that two or more events have been executed ("YES" in step S604), the CPU 11 determines whether or not to execute the next event (step S605). For example, the CPU 11 may determine to execute the next event when the user performs an operation of instructing to execute the next event. When a predetermined number of events have already been executed, the CPU 11 may determine not to execute the next event. The CPU 11 may determine not to execute the next event when the user performs an operation of instructing the end of the event training.

When determining that the next event is to be executed ("YES" in step S605), or when determining that two or more events have not been executed yet in step S604 ("NO" in step S604), the CPU 11 selects an event to be executed next from among the plurality of events included in the event management data 133 based on the evaluation result of the activity state in the completed event, and acquires the evaluation condition of the selected event (step S606). A method for selecting an event to be performed next is not particularly limited, but for example, an activity state that the user is not good at may be specified based on the evaluation result of the completed event, and an event for which the activity state is an evaluation target may be selected as the event to be performed next. When the processing of step S606 ends, the CPU 11 returns the processing to step S603 and executes the event execution processing for the selected event.

When determining that the next event is not to be executed ("NO" in step S605), the CPU 11 ends the event training execution processing.

### (Modification 7)

In the above embodiment, the case of relatively evaluating the activity states of the plurality of users has been exemplified, but the method for evaluating the activity state is not limited to the relative evaluation but may be an absolute evaluation based on the evaluation value of the activity state of a certain user. For example, one terminal device 20 and one wearable device 30 used by a certain user may constitute the activity support system 1, and the terminal device 20 may perform the absolute evaluation of the activity state of the user based on the activity index acquired by the wearable device 30. In this case, the CPU 21 of the terminal device 20 corresponds to a "computer". Each of participating users for the plurality of events executed in this case is one user who uses the terminal device 20 and the wearable device 30. A method for setting the evaluation item in each of the plurality of events can be similar to the above embodiment and Modifications 1 to 6.

When the terminal device 20 includes a sensor unit, the wearable device 30 may be omitted in the activity support system 1, and the activity support system 1 may be formed only of the terminal device 20. In this case, the terminal device 20 acquires the activity index of the activity of the user, and the CPU 21 of the terminal device 20 evaluates the activity state based on the activity index.

When the wearable device 30 can execute the function executed by the terminal device 20 in the above embodiment, the terminal device 20 may be omitted in the activity support system 1, and the activity support system 1 may be formed only of the wearable device 30. In this case, the wearable device 30 acquires the activity index of the activity of the user, and the CPU 31 of the wearable device 30 evaluates the activity state based on the activity index. The CPU 31 of the wearable device 30 corresponds to a "computer".

### (Modification 8)

In the above embodiment, the example in which the event training (i.e., the first event that is the first event of the event training) is executed irregularly in response to the request of the user has been described, but the present invention is not limited thereto, and the event training (first event) may be executed periodically, for example, at a predetermined frequency. The present modification may be combined with at least one of Modifications 1 to 7.

### (Modification 9)

The evaluation of the activity state is not limited to the rank in the ranking of the activity state of the participating user. For example, the evaluation of the activity state may be a representation of the position of each user in the ranking of the activity state of the participating user in proportion (e.g., the highest percentage is 100%, and the lowest percentage is 0%). The evaluation value itself of the activity state may be presented to the user as the evaluation of the activity state. The present modification may be combined with at least one of Modifications 1 to 8.

### (Modification 10)

In each event of the event training, information related to the event may be able to be shared between the participating users. For example, an information sharing means such as a website or an SNS may be prepared so that any participating user can write in the information sharing means, and the information written in the information sharing means can be browsed by other participating users. Such an information sharing means may be provided for each event. According to this, in the event in which the specific activity state is evaluated, it is possible to share information useful for improving the skill, such as what each user is conscious of to raise the evaluation. As a result, it is possible to enhance the motivation of the training and to more effectively improve the skill. The present modification may be combined with at least one of Modifications 1 to 9.

### (Modification 11)

A part where the wearable device 30 is worn is not limited to the waist. For example, the wearable device 30 may be a wrist-type terminal used by being worn on the wrist of the user. Here, the wearable device 30 worn on the waist and the wearable device 30 worn on the wrist may have different activity indexes that can be acquired. Thus, the event in which the user participates may be determined based on the type of the wearable device 30 worn by the user in accordance with the wearing part. That is, an evaluable activity state may be specified based on the detection result of the wearable device 30 worn by the user, and an event in which the activity state is set as an evaluation target may be selected as an event in which the user participates. For example, when the wearable device 30 worn on the wrist is used, the "arm swing magnitude" as the activity state can be evaluated from the detection result of the wearable device 30, and hence an event in which the activity state of the "arm swing magnitude" is the evaluation item may be selected. The present modification may be combined with at least one of Modifications 1 to 10. An ankle, a knee, a head, or the like can be selected as a part in addition to the waist or the wrist.

### (Modification 12)

At least one of the plurality of events that make up the event training may be executed in a virtual space. For example, the user runs using a machine such as a treadmill, and causes the display unit 24 of the terminal device 20, a display unit of a head-mounted display worn by the user, a display unit provided in the machine, or the like to display the image of the viewpoint of the user running on the running path in the virtual space in accordance with the running state (distance, speed, etc.) on the machine. On the running path in the virtual space, a virtual image of another participating user running on the running path may be displayed in accordance with the running state of another participating user. The present modification may be combined with at least one of Modifications 1 to 11.

### <Effects>

As described above, the information processing method according to the present embodiment causes the CPU 11 as the computer to execute the first event for evaluating the first activity state of the user when the user as the subject is running (first activity) (step S105 (first time) in Fig. 8), to determine whether the user has received the evaluation in the first event (step S208 in Fig. 9), and to execute the second event for evaluating the second activity state, which is the activity state of the user when the user is running (second activity) and is different from the first activity state, at least on the condition that it is determined that the user has received the evaluation in the first event (step S105 after step S107 in Fig. 8). As thus described, the second event at least on the condition that it is determined that the user has received the evaluation in the first event, whereby at least two events can be executed in the order intended by an event promoter. The user can receive evaluations of different activity states in these at least two events. Therefore, the movement of the activity (running form, etc.) can be improved from multiple perspectives with reference to these evaluations, thus improving the skill related to the activity efficiently and comprehensively.

The information processing method according to Modification 6 causes the CPU 11 to select the second event from a plurality of events for evaluating different activity states based on the evaluation result in the first event (step S606 in Fig. 18). As a result, the content of the second event can be flexibly changed such that the skill is effectively improved in accordance with the state of the skill related to the activity of the user.

The information processing method causes the CPU 11 to execute the first event, thereby acquiring the first activity index detected by the wearable device 30 when the user performs the first activity with the wearable device 30 worn on the body (step S203 in Fig. 9), and evaluating the first activity state based on the acquired first activity index (steps S205 and S206 in Fig. 9). The information processing method causes the CPU 11 to execute the second event, thereby acquiring the second activity index detected by the wearable device 30 when the user performs the second activity with the wearable device 30 worn on the body (step S203 in Fig. 9), and evaluating the second activity state based on the acquired second activity index (steps S205 and S206 in Fig. 9). As described above, according to the method for evaluating the activity state based on the activity index that can be acquired by the wearable device 30 worn by the user, the activity state can be evaluated without using a large-scale device. In addition, the activity state can be accurately evaluated in accordance with the actual movement of the user's body.

As described in Modification 8, the first event may be executed regularly or irregularly. By periodically performing the event training including the first event, it is possible to continuously improve the skill related to activity. In addition, training can be made a habit. On the other hand, by adopting a mode in which event training including the first event can be performed irregularly, training can be performed by selecting a time convenient for the user.

The subject is a participating user including a plurality of users different from each other, and the first activity state of each of the participating users performing the first activity is relatively evaluated in the first event, and the second activity state of each of the participating users performing the second activity is relatively evaluated in the second event. As a result, the user can intuitively grasp the state of his/her skill in comparison with other participating users. Further, an element of competition with other participating users is added, so that motivation for activity can be increased.

In Modification 2, the first activity and the second activity are running (the same certain activity), and the first activity state and the second activity state are activity states in which the condition related to the first activity state and the condition related to the second activity state are in a trade-off relationship with each other. As a result, it is possible to efficiently and comprehensively improve the skill related to activity such that the evaluation value naturally improves for two activity states in which it is not easy to improve the evaluation value at a time.

The first activity state is an activity state representing a burden on the user's body due to running. Thus, the skill for reducing the burden on the body can be improved with priority, enabling the remaining events to be performed with less burden on the body and less chance of injury. As a result, the event period can be lengthened, effectively improving the skill related to activity.

The first activity state or the second activity state includes at least one of the left-right symmetry of the activity of the user, the posture of the user, and the ground contact state of the user. This makes it possible to efficiently and comprehensively improve particularly important skills in running.

The first activity state or the second activity state is derived based on the activity index, and the activity index includes at least one of a pitch, a stride, a ground contact time, and a ground contact time rate of the user performing running, a vertical movement, a left-right movement, a front-back movement, and an impulse in the body of the user, a rotation angle in each of axes of a pelvis of the user, sinking, a propulsion magnitude, a propulsion direction, a propulsion timing, a brake magnitude, a brake distance, a swing-backward time and swing-forward time of a swinging leg, a placement time, and stiffness. These activity indexes can be derived based on the detection result of the sensor unit 34 of the wearable device 30 worn by the user, so that it is possible to derive the activity index and evaluate the activity state without using a large-scale device.

In Modification 1, the first activity and the second activity are running (the same certain activity), and before the CPU 11 is caused to execute the first event, the CPU 11 is caused to execute a comprehensive evaluation event for comprehensively evaluating a plurality of mutually different activity states including the first activity state and the second activity state of the user performing a certain activity, determine whether the user has received the evaluation in the comprehensive evaluation event, and execute the first event at least on a condition that it is determined that the user has received the evaluation in the comprehensive evaluation event. By first comprehensively evaluating the activity state in the comprehensive evaluation event as thus described, the user can grasp the comprehensive skill state at the start of the event training.

In Modification 1, the CPU 11 is caused to determine whether the user has received the evaluation in the second event, and to execute the comprehensive evaluation event at least on the condition that it is determined that the user has received the evaluation in the second event. As a result, the user can grasp how much the comprehensive activity skill of the user has been improved through the first event and the second event by comparison with the evaluation in the first comprehensive evaluation event.

The information processing method causes the CPU 11 to execute at least one of the first event and the second event online or in a virtual space (Modification 12). By executing the event online, the user can participate in the event at an arbitrary position and can receive a relative evaluation with other participating users who are not actually running together. By executing the event in the virtual space, the training can be performed as if the user is running on the running path without actually running on the running path. This can enhance motivation for activity.

The program 131 according to the present embodiment causes the CPU 11 as the computer to execute the first event for evaluating the first activity state of the user when the user as the subject is performing the first activity (step S105 (first time) in Fig. 8), to determine whether the user has received the evaluation in the first event (step S208 in Fig. 9), and to execute the second event for evaluating the second activity state, which is the activity state of the user when the user is performing the second activity and is different from the first activity state, at least on the condition that it is determined that the user has received the evaluation in the first event (step S105 after step S107 in Fig. 8). As thus described, the second event at least on the condition that it is determined that the user has received the evaluation in the first event, whereby at least two events can be executed in the order intended by an event promoter. The user can receive evaluations of different activity states in these at least two events. Therefore, the movement of the activity (running form, etc.) can be improved from multiple perspectives with reference to these evaluations, thus improving the skill related to the activity efficiently and comprehensively.

Furthermore, the activity support system 1 as an information processing system according to the present embodiment includes the CPU 11 as the computer, in which the CPU 11 executes the first event for evaluating the first activity state of the user when the user as the subject is performing the first activity (step S105 (first time) in Fig. 8), determines whether the user has received the evaluation in the first event (step S208 in Fig. 9), and executes the second event for evaluating the second activity state, which is the activity state of the user when the user is performing the second activity and is different from the first activity state, at least on the condition that it is determined that the user has received the evaluation in the first event (step S105 after step S107 in Fig. 8). As thus described, the second event at least on the condition that it is determined that the user has received the evaluation in the first event, whereby at least two events can be executed in the order intended by an event promoter. The user can receive evaluations of different activity states in these at least two events. Therefore, the movement of the activity (running form, etc.) can be improved from multiple perspectives with reference to these evaluations, thus improving the skill related to the activity efficiently and comprehensively.

### <Others>

Note that the descriptions in the above embodiment are examples of the information processing method, the program, and the information processing system according to the present invention, and are not limited thereto.

For example, any of the terminal devices 20 may execute the processing performed by the server 10 as the information processing device in the above embodiment. In this case, the CPU 21 of the terminal device 20 corresponds to a "computer". In the above embodiment, the processing performed by the server 10 as the information processing device may be executed by any of the wearable devices 30. In this case, the CPU 31 of the wearable device 30 corresponds to a "computer".

The terminal device 20 and the wearable device 30 may be integrated. For example, when the terminal device 20 includes a sensor unit, the terminal device 20 may detect the movement of the body due to the activity of the user, and the wearable device 30 may be omitted. In this case, the activity support system 1 includes the server 10 and a plurality of terminal devices 20. When the wearable device 30 can execute the function executed by the terminal device 20 in the above embodiment, the terminal device 20 may be omitted. In this case, the activity support system 1 includes the server 10 and a plurality of wearable devices 30.

An example in which the CPU 31 of the wearable device 30 derives the activity index based on the detection result of the sensor unit 34 has been described, but the invention is not limited thereto. For example, the detection result of the sensor unit 34 may be transmitted to the terminal device 20, and the CPU 21 of the terminal device 20 may derive the activity index, or the detection result of the sensor unit 34 may be transmitted to the server 10, and the CPU 11 of the server 10 may derive the activity index.

An example in which the CPU 11 of the server 10 derives the evaluation value of the activity state based on the received activity index has been described, but the present invention is not limited thereto. For example, the CPU 21 of the terminal device 20 may derive the evaluation value of the activity state based on the activity index received from the wearable device 30 and transmit the derivation result to the server 10. The CPU 31 of the wearable device 30 may derive the evaluation value of the activity state based on the activity index, and the evaluation result may be directly transmitted to the server 10 via the terminal device 20 or to the server 10 without the terminal device 20.

Although running and walking have been exemplified as each of the first activity and the second activity performed by the user as the subject, each of the first activity and the second activity is not limited thereto but may be, for example, riding a bicycle or the like. In addition, each of the first activity and the second activity is not necessarily limited to the activity involving movement but may be gymnastic activity or muscle strength training.

The subject for evaluating the activity state is not limited to a person as long as the subject can perform the activity. For example, the subject for evaluating the activity state may be an animal, a robot, or the like.

In the above description, an example in which the storage units 13, 23, 33 are used as the computer-readable medium of the program according to the present invention has been disclosed, but the present invention is not limited to this example. As another computer-readable medium, an information recording medium such as an HDD, an SSD, a flash memory, or a CD-ROM can be applied. A carrier wave is also applied to the present invention as a medium for providing data of a program according to the present invention through a communication line.

## Claims

1. A computer-implemented method comprising:
executing a first event for evaluating a first activity state of a subject when the subject is performing a first activity by acquiring a first activity index detected by a sensor when a subject is performing the first activity with the sensor worn on a body, and by evaluating the first activity state based on the acquired first activity index;
determining that the subject received the evaluation in the first event when an event terminable condition of the first event is satisfied; and
executing a second event for evaluating a second activity state different from the first activity state, the second activity state being an activity state of the subject when the subject is performing a second activity, at least on a condition that it is determined that the subject received the evaluation in the first event, by acquiring a second activity index detected by the sensor when the subject is performing the second activity with the sensor worn on the body, and by evaluating the second activity state based on the acquired second activity index.

2. The computer-implemented method according to claim 1, wherein the method comprises
selecting the second event from a plurality of events for evaluating different activity states based on an evaluation result in the first event.

3. The computer-implemented method according to claim 1 or 2, wherein
the first event is executed regularly or irregularly.

4. The computer-implemented method according to any one of claims 1 to 3, wherein
the subject is a plurality of different subjects,
the first activity state of each of the plurality of subjects performing the first activity is relatively evaluated in the first event, and
the second activity state of each of the plurality of subjects performing the second activity is relatively evaluated in the second event.

5. The computer-implemented method according to any one of claims 1 to 4, wherein
the first activity and the second activity are the same certain activity, and
the first activity state and the second activity state are activity states in which a condition related to the first activity state and a condition related to the second activity state are in a trade-off relationship with each other.

6. The computer-implemented method according to any one of claims 1 to 5, wherein
the first activity state is an activity state representing a burden on the body of the subject due to the certain activity.

7. The computer-implemented method according to claim 5 or 6, wherein
the certain activity is walking or running, and
the first activity state or the second activity state includes at least one of left-right symmetry of the activity of the subject, a posture of the subject, and a ground contact state of the subject.

8. The computer-implemented method according to claim 7, wherein
the first activity state or the second activity state is derived based on an activity index, and the activity index includes at least one of a pitch, a stride, a ground contact time, and a ground contact time rate of the subject performing the walking or the running, a vertical movement, a left-right movement, a front-back movement, and an impulse in the body of the subject, a rotation angle in each of axes of a pelvis of the subject, sinking, a propulsion magnitude, a propulsion direction, a propulsion timing, a brake magnitude, a brake distance, a swing-backward time and swing-forward time of a swinging leg, a placement time, and stiffness.

9. The computer-implemented method according to any one of claims 1 to 8, wherein
the first activity and the second activity are the same certain activity, and wherein the method comprises
executing a step of comprehensively evaluating a plurality of different activity states that include the first activity state and the second activity state of the subject performing the certain activity before executing the first event; and
executing the first event at least on a condition that the step is executed.

10. The computer-implemented method according to claim 9, wherein the method comprises
determining that the subject received the evaluation in the second event when an event terminable condition of the second event is satisfied.

11. The computer-implemented method according to any one of claims 1 to 10, wherein the method comprises
executing at least one of the first event and the second event online or in a virtual space.

12. A non-transitory computer-readable recording medium that records a program executable by at least one processor (11, 21, 31) of a computer, the program causes the at least one processor (11, 21, 31) to carry out steps of:
executing a first event for evaluating a first activity state of a subject when the subject is performing a first activity by acquiring a first activity index detected by a sensor when a subject is performing the first activity with the sensor worn on a body, and by evaluating the first activity state based on the acquired first activity index;
determining that the subject received the evaluation in the first event when an event terminable condition of the first event is satisfied; and
executing a second event for evaluating a second activity state different from the first activity state, the second activity state being an activity state of the subject when the subject is performing a second activity, at least on a condition that it is determined that the subject received the evaluation in the first event, by acquiring a second activity index detected by the sensor when the subject is performing the second activity with the sensor worn on the body, and by evaluating the second activity state based on the acquired second activity index.

13. An information processing system (1) comprising a computer configured to:
execute a first event for evaluating a first activity state of a subject when the subject is performing a first activity by acquiring a first activity index detected by a sensor when a subject is performing the first activity with the sensor worn on a body, and by evaluating the first activity state based on the acquired first activity index;
determine that the subject received the evaluation in the first event when an event terminable condition of the first event is satisfied; and
execute a second event for evaluating a second activity state different from the first activity state, the second activity state being an activity state of the subject when the subject is performing a second activity, at least on a condition that it is determined that the subject received the evaluation in the first event, by acquiring a second activity index detected by the sensor when the subject is performing the second activity with the sensor worn on the body, and by evaluating the second activity state based on the acquired second activity index.

## Patentansprüche

1. Computerimplementiertes Verfahren, umfassend:
Ausführen eines ersten Ereignisses zum Bewerten eines ersten Aktivitätszustands eines Subjekts, wenn das Subjekt eine erste Aktivität durch Erfassen eines ersten Aktivitätsindexes, der von einem Sensor detektiert wird, wenn ein Subjekt die erste Aktivität mit dem an einem Körper getragenen Sensor ausführt, und durch Bewerten des ersten Aktivitätszustands basierend auf dem erfassten ersten Aktivitätsindex;
Bestimmen, dass das Subjekt die Bewertung in dem ersten Ereignis empfangen hat, wenn eine Ereignisbeendigungsbedingung des ersten Ereignisses erfüllt ist; und
Ausführen eines zweiten Ereignisses zum Bewerten eines zweiten Aktivitätszustands, der sich von dem ersten Aktivitätszustand unterscheidet, wobei der zweite Aktivitätszustand ein Aktivitätszustand des Subjekts ist, wenn das Subjekt eine zweite Aktivität durchführt, mindestens unter einer Bedingung, dass bestimmt wird, dass das Subjekt die Bewertung in dem ersten Ereignis empfangen hat, durch Erfassen eines zweiten Aktivitätsindex, der von dem Sensor detektiert wird, wenn das Subjekt die zweite Aktivität mit dem am Körper getragenen Sensor durchführt, und durch Bewerten des zweiten Aktivitätszustands basierend auf dem erfassten zweiten Aktivitätsindex.

2. Computerimplementiertes Verfahren nach Anspruch 1, wobei das Verfahren Folgendes umfasst
Auswählen des zweiten Ereignisses aus einer Vielzahl von Ereignissen zum Bewerten verschiedener Aktivitätszustände basierend auf einem Bewertungsergebnis in dem ersten Ereignis.

3. Computerimplementiertes Verfahren nach Anspruch 1 oder 2, wobei
das erste Ereignis regelmäßig oder unregelmäßig ausgeführt wird.

4. Computerimplementiertes Verfahren nach einem der Ansprüche 1 bis 3, wobei
das Subjekt eine Vielzahl von verschiedenen Subjekten ist,
der erste Aktivitätszustand von jedem der Vielzahl von Subjekten, die die erste Aktivität durchführen, in dem ersten Ereignis relativ bewertet wird, und
der zweite Aktivitätszustand von jedem der Vielzahl von Subjekten, die die zweite Aktivität durchführen, relativ in dem zweiten Ereignis bewertet wird.

5. Computerimplementiertes Verfahren nach einem der Ansprüche 1 bis 4, wobei
die erste Aktivität und die zweite Aktivität die gleiche bestimmte Aktivität sind, und
der erste Aktivitätszustand und der zweite Aktivitätszustand Aktivitätszustände sind, in denen eine Bedingung, die den ersten Aktivitätszustand betrifft, und eine Bedingung, die den zweiten Aktivitätszustand betrifft, in einer Trade-off-Beziehung zueinander sind.

6. Computerimplementiertes Verfahren nach einem der Ansprüche 1 bis 5, wobei
der erste Aktivitätszustand ein Aktivitätszustand ist, der eine Belastung für den Körper des Subjekts aufgrund der bestimmten Aktivität repräsentiert.

7. Computerimplementiertes Verfahren nach Anspruch 5 oder 6, wobei
die bestimmte Aktivität Gehen oder Laufen ist, und
der erste Aktivitätszustand oder der zweite Aktivitätszustand mindestens eines aus einer Links-Rechts-Symmetrie der Aktivität des Subjekts, einer Körperhaltung des Subjekts und einem Bodenkontaktzustand des Subjekts einschließt.

8. Computerimplementiertes Verfahren nach Anspruch 7, wobei
der erste Aktivitätszustand oder der zweite Aktivitätszustand basierend auf einem Aktivitätsindex abgeleitet wird, und der Aktivitätsindex mindestens eines aus einer Schrittlänge, einem Schritt, einer Bodenkontaktzeit und einer Bodenkontaktzeitrate des Subjekts, das das Gehen oder das Laufen durchführt, einer vertikalen Bewegung, einer Links-Rechts-Bewegung, einer Vorwärts-RückwärtsBewegung, und einem Impuls im Körper des Subjekts, einem Drehwinkel in jeder der Achsen eines Beckens des Subjekts, einem Absinken, einer Vortriebsgrößenordnung, einer Vortriebsrichtung, einem Vortriebstiming, einer Bremsgrößenordnung, einem Bremsabstand, einer Rückschwingzeit und einer Vorschwingzeit eines schwingenden Beins, einer Platzierungszeit und einer Steifigkeit einschließt.

9. Computerimplementiertes Verfahren nach einem der Ansprüche 1 bis 8, wobei
die erste Aktivität und die zweite Aktivität die gleiche bestimmte Aktivität sind, und wobei das Verfahren Folgendes umfasst
Ausführen eines Schritts des umfassenden Bewertens einer Vielzahl von verschiedenen Aktivitätszuständen, die den ersten Aktivitätszustand und den zweiten Aktivitätszustand des Subjekts einschließen, das die bestimmte Aktivität durchführt, bevor das erste Ereignis ausgeführt wird; und
Ausführen des ersten Ereignisses mindestens unter einer Bedingung, dass der Schritt ausgeführt wird.

10. Computerimplementiertes Verfahren nach Anspruch 9, wobei das Verfahren Folgendes umfasst
Bestimmen, dass das Subjekt die Bewertung in dem zweiten Ereignis empfangen hat, wenn eine Ereignisbeendigungsbedingung des zweiten Ereignisses erfüllt ist.

11. Computerimplementiertes Verfahren nach einem der Ansprüche 1 bis 10, wobei das Verfahren Folgendes umfasst
Ausführen mindestens eines aus dem ersten Ereignis und dem zweiten Ereignis online oder in einem virtuellen Raum.

12. Nicht transitorisches, computerlesbares Aufzeichnungsmedium, das ein Programm aufzeichnet, das von mindestens einem Prozessor **(11,** 21, 31) eines Computers ausführbar ist, wobei das Programm den mindestens einen Prozessor **(11,** 21, 31) veranlasst, die folgenden Schritte auszuführen:
Ausführen eines ersten Ereignisses zum Bewerten eines ersten Aktivitätszustands eines Subjekts, wenn das Subjekt eine erste Aktivität durch Erfassen eines ersten Aktivitätsindexes, der von einem Sensor detektiert wird, wenn ein Subjekt die erste Aktivität mit dem an einem Körper getragenen Sensor durchführt, und durch Bewerten des ersten Aktivitätszustands basierend auf dem erfassten ersten Aktivitätsindex;
Bestimmen, dass das Subjekt die Bewertung in dem ersten Ereignis empfangen hat, wenn eine Ereignisbeendigungsbedingung des ersten Ereignisses erfüllt ist; und
Ausführen eines zweiten Ereignisses zum Bewerten eines zweiten Aktivitätszustands, der sich von dem ersten Aktivitätszustand unterscheidet, wobei der zweite Aktivitätszustand ein Aktivitätszustand des Subjekts ist, wenn das Subjekt eine zweite Aktivität durchführt, mindestens unter einer Bedingung, dass bestimmt wird, dass das Subjekt die Bewertung in dem ersten Ereignis empfangen hat, durch Erfassen eines zweiten Aktivitätsindex, der von dem Sensor detektiert wird, wenn das Subjekt die zweite Aktivität mit dem am Körper getragenen Sensor durchführt, und durch Bewerten des zweiten Aktivitätszustands basierend auf dem erfassten zweiten Aktivitätsindex.

13. Informationsverarbeitungssystem (1), umfassend einen Computer, konfiguriert zum:
Ausführen eines ersten Ereignisses zum Bewerten eines ersten Aktivitätszustands eines Subjekts, wenn das Subjekt eine erste Aktivität durch Erfassen eines ersten Aktivitätsindexes, der von einem Sensor detektiert wird, wenn ein Subjekt die erste Aktivität mit dem an einem Körper getragenen Sensor durchführt, und durch Bewerten des ersten Aktivitätszustands basierend auf dem erfassten ersten Aktivitätsindex;
Bestimmen, dass das Subjekt die Bewertung in dem ersten Ereignis empfangen hat, wenn eine Ereignisbeendigungsbedingung des ersten Ereignisses erfüllt ist; und
Ausführen eines zweiten Ereignisses zum Bewerten eines zweiten Aktivitätszustands, der sich von dem ersten Aktivitätszustand unterscheidet, wobei der zweite Aktivitätszustand ein Aktivitätszustand des Subjekts ist, wenn das Subjekt eine zweite Aktivität durchführt, mindestens unter einer Bedingung, dass bestimmt wird, dass das Subjekt die Bewertung in dem ersten Ereignis empfangen hat, durch Erfassen eines zweiten Aktivitätsindex, der von dem Sensor detektiert wird, wenn das Subjekt die zweite Aktivität mit dem am Körper getragenen Sensor durchführt, und durch Bewerten des zweiten Aktivitätszustands basierend auf dem erfassten zweiten Aktivitätsindex.

## Revendications

1. Procédé implémenté sur ordinateur, comprenant les étapes consistant à :
exécuter un premier évènement pour évaluer un premier état d'activité d'un sujet lorsque le sujet accomplit une première activité, en acquérant un premier indice d'activité détecté par un capteur lorsqu'un sujet accomplit la première activité avec le capteur porté sur un corps, et en évaluant le premier état d'activité sur la base du premier indice d'activité acquis ;
déterminer que le sujet a reçu l'évaluation lors du premier évènement lorsqu'une condition du premier évènement, permettant de terminer l'évènement, est remplie ; et
exécuter un second évènement pour évaluer un second état d'activité différent du premier état d'activité, le second état d'activité étant un état d'activité du sujet lorsque le sujet accomplit une seconde activité, au moins à condition qu'il soit déterminé que le sujet a reçu l'évaluation lors du premier évènement, en acquérant un second indice d'activité détecté par le capteur lorsque le sujet accomplit la seconde activité avec le capteur porté sur le corps, et en évaluant le second état d'activité sur la base du second indice d'activité acquis.

2. Procédé implémenté sur ordinateur selon la revendication 1, le procédé comprenant
le fait de sélectionner le second évènement parmi une pluralité d'évènements pour évaluer différents états d'activité sur la base d'un résultat d'évaluation lors du premier évènement.

3. Procédé implémenté sur ordinateur selon la revendication 1 ou 2, dans lequel le premier évènement est exécuté de façon régulière ou irrégulière.

4. Procédé implémenté sur ordinateur selon l'une quelconque des revendications 1 à 3, dans lequel
le sujet est une pluralité de sujets différents,
le premier état d'activité de chacun parmi la pluralité de sujets accomplissant la première activité est évalué relativement lors du premier évènement, et
le second état d'activité de chacun parmi la pluralité de sujets accomplissant la seconde activité est évalué relativement lors du second évènement.

5. Procédé implémenté sur ordinateur selon l'une quelconque des revendications 1 à 4, dans lequel
la première activité et la seconde activité consistent en la même activité donnée, et
le premier état d'activité et le second état d'activité sont des états d'activité dans lesquels une condition liée au premier état d'activité et une condition liée au second état d'activité se trouvent dans une relation de compromis l'une avec l'autre.

6. Procédé implémenté sur ordinateur selon l'une quelconque des revendications 1 à 5, dans lequel
le premier état d'activité est un état d'activité représentant une sollicitation exercée sur le corps du sujet, due à l'activité donnée.

7. Procédé implémenté sur ordinateur selon la revendication 5 ou 6, dans lequel
l'activité donnée est la marche ou la course, et
le premier état d'activité ou le second état d'activité inclut au moins un parmi la symétrie gauche-droite de l'activité du sujet, une posture du sujet, et un état de contact du sujet avec le sol.

8. Procédé implémenté sur ordinateur selon la revendication 7, dans lequel
le premier état d'activité ou le second état d'activité est déduit sur la base d'un indice d'activité, et l'indice d'activité inclut au moins un parmi une cadence, une foulée, un temps de contact avec le sol, et un ratio de temps de contact avec le sol du sujet accomplissant la marche ou la course, un mouvement vertical, un mouvement gauche-droite, un mouvement avant-arrière et une impulsion dans le corps du sujet, un angle de rotation selon chacun des axes d'un bassin du sujet, un affaissement, une amplitude de propulsion, une direction de propulsion, un positionnement temporel de propulsion, une intensité de freinage, une distance de freinage, un temps de balancement vers l'arrière et un temps de balancement vers l'avant d'une jambe en balancement, un temps de placement, et une raideur.

9. Procédé implémenté sur ordinateur selon l'une quelconque des revendications 1 à 8, dans lequel
la première activité et la seconde activité consistent en la même activité donnée, et le procédé comprenant :
le fait d'exécuter une étape d'évaluation exhaustive d'une pluralité d'états d'activité différents qui incluent le premier état d'activité et le second état d'activité du sujet accomplissant l'activité donnée, avant d'exécuter le premier événement ; et
le fait d'exécuter le premier événement au moins à condition que l'étape soit exécutée.

10. Procédé implémenté sur ordinateur selon la revendication 9, le procédé comprenant l'étape consistant à
déterminer que le sujet a reçu l'évaluation lors du second événement lorsqu'une condition du second évènement, permettant de terminer l'évènement, est remplie.

11. Procédé implémenté sur ordinateur selon l'une quelconque des revendications 1 à 10, le procédé comprenant
le fait d'exécuter au moins un parmi le premier évènement et le second évènement en ligne ou dans un espace virtuel.

12. Support d'enregistrement non transitoire lisible par ordinateur, sur lequel est enregistré un programme exécutable par au moins un processeur (11, 21, 31) d'un ordinateur, le programme amène ledit au moins un processeur (11, 21, 31) à mettre en œuvre les étapes consistant à :
exécuter un premier évènement pour évaluer un premier état d'activité d'un sujet lorsque le sujet accomplit une première activité, en acquérant un premier indice d'activité détecté par un capteur lorsqu'un sujet accomplit la première activité avec le capteur porté sur un corps, et en évaluant le premier état d'activité sur la base du premier indice d'activité acquis ;
déterminer que le sujet a reçu l'évaluation lors du premier évènement lorsqu'une condition du premier évènement, permettant de terminer l'évènement, est remplie ; et
exécuter un second évènement pour évaluer un second état d'activité différent du premier état d'activité, le second état d'activité étant un état d'activité du sujet lorsque le sujet accomplit une seconde activité, au moins à condition qu'il soit déterminé que le sujet a reçu l'évaluation lors du premier évènement, en acquérant un second indice d'activité détecté par le capteur lorsque le sujet accomplit la seconde activité avec le capteur porté sur le corps, et en évaluant le second état d'activité sur la base du second indice d'activité acquis.

13. Système de traitement de l'information (1) comprenant un ordinateur configuré pour :
exécuter un premier évènement pour évaluer un premier état d'activité d'un sujet lorsque le sujet accomplit une première activité, en acquérant un premier indice d'activité détecté par un capteur lorsqu'un sujet accomplit la première activité avec le capteur porté sur un corps, et en évaluant le premier état d'activité sur la base du premier indice d'activité acquis ;
déterminer que le sujet a reçu l'évaluation lors du premier évènement lorsqu'une condition du premier évènement, permettant de terminer l'évènement, est remplie ; et
exécuter un second évènement pour évaluer un second état d'activité différent du premier état d'activité, le second état d'activité étant un état d'activité du sujet lorsque le sujet accomplit une seconde activité, au moins à condition qu'il soit déterminé que le sujet a reçu l'évaluation lors du premier évènement, en acquérant un second indice d'activité détecté par le capteur lorsque le sujet accomplit la seconde activité avec le capteur porté sur le corps, et en évaluant le second état d'activité sur la base du second indice d'activité acquis.
